# EUROPEAN PATENT SPECIFICATION

(11) **EP 0 210 189 B1**
(45) Date of publication and mention of the grant of the patent: **07.12.1994**
(21) Application number: 86900477.0
(22) Date of filing: 10.12.1985
(51) Int. Cl.: C07K 7/10, A61K 37/02, C12N 15/00

(54) **RECOMBINANT ALVEOLAR SURFACTANT PROTEIN**
REKOMBINANTES ALVEOLARES OBERFLÄCHENAKTIVES PROTEIN
PROTEINE TENSIO-ACTIVE ALVEOLAIRE RECOMBINANTE

(30) Priority: 11.12.1984 US 680358
(43) Date of publication of application: 04.02.1987
(73) Proprietor: SCIOS NOVA INC., Mountain View, CA 94043 (US)
(72) Inventor: SCHILLING, James, W., Jr., Palo Alto, CA 94301 (US); WHITE, Robert, T., Fremont, CA 94538 (US); CORDELL, Barbara, San Francisco, CA 94109 (US); BENSON, Bradley, J., San Francisco, CA 94127 (US)
(74) Representative: Goldin, Douglas Michael
(86) International application number: PCT/US85/02445
(87) International publication number: WO 86/03408

(56) References cited:
- US-A- 4 506 013
- US-A- 4 562 003
- CHEMICAL ABSTRACTS, vol. 101, no. 17, 22nd October 1984, page 347, abstract no. 147230n, Columbus, Ohio, US; S.L. KATYAL et al.: "Analysis of pulmonary surfactant apoproteins by isoelectric focusing"
- CHEMICAL ABSTRACTS, vol. 95, no. 23, 7th December 1981, page 259, abstract no. 199389y, Columbus, Ohio, US; S.L. KATYAL et al.: "Analysis of pulmonary surfactant apoproteins by electrophoresis"
- PROC. NATL. ACAD. SCI. USA, vol. 82, October 1985, pages 6379-6383; B. BENSON et al.: "Structure of canine pulmonary surfactant apoprotein: cDNA and complete amino acid sequence"
- NATURE, vol. 317, 26th September 1985, pages 361-363; R. TYLER WHITE et al.: "Isolation and characterization of the human pulmonary surfactant apoprotein gene"
- Chemical Abstracts, vol. 103, 1985, abstract no. 85619b, Balis et al.
- Chemical Abstracts, vol. 99, 1983, abstract no. 155807, Maki
- Chemical Abstracts, vol. 102, 1985, 102; 217062f, Whitsett et al.
- Biological Abstracts, vol. 75, 1982, abstract no. 14696, Shelley et al.
- Biological Abstracts, vol. 80, 1985, abstract no. 63225
- Chemical Abstracts, vol. 101, 1984, abstract no. 2279634, Balis et al.
- Chemical Abstracts, vol. 91, 1979, abxtract no. 190210t, Bruni et al.
- Biochim. Biophys. Acta, 791 81984 9, pp. 226-238, Phelps, D.S. et al.
- Biochim. Biophys. Acta, 665(1981), pp. 442-453, Sueishi, K. et al.
- Am. J. Physiol., vol. 224, no. 4 (1973), pp. 788-795, King, R.J. et al.

## Description

### Technical Field

The invention relates to the field of recombinant protein production. More specifically it relates to the production of alveolar surfactant protein (ASP) which is useful in the management of certain respiratory diseases.

### Background Art

The human lung is composed of a large number of small sacs or alveoli in which gases are exchanged between the blood and the air spaces of the lung. In healthy individuals, this exchange is mediated by the presence of a protein containing surfactant complex which is synthesized in the microsomal membranes of type II alveolar cells. In the absence of adequate levels of this complex, a lung cannot properly function--i.e., the alveoli collapse during exhalation, and cannot be subsequently re-inflated by inhaling. Thus, the untreated inability to synthesize this complex may result in death or in severe physical damage.

The best documented instance of inadequate surfactant complex levels occurs in premature infants and infants born after complicated pregnancies, and is widely known as respiratory distress syndrome (RDS). A widely publicized form of this syndrome has been designated hyaline membrane disease, or idiopathic RDS. RDS is currently the leading cause of infant mortality and morbidity in the United States and in other developed countries, and substantial efforts have been directed to diagnosis and treatment. Current treatment has focused on mechanical (pressure) ventilation which, at best, is an invasive stop-gap measure that often results in damage to the lung and other deleterious side effects, including complications such as bronchopulmonary dysplasia, interstitial emphysema and pneumothorax. Mental retardation has also resulted on occasion when this treatment was used (Hallman, M., et al, Pediatric Clinics of North America (1982) 29:1057-1075).

Limited attempts have been made to treat the syndrome by surfactant substitution. This would be a method of choice, as, in general, only one administration is required, and the potential for damage is reduced. For example, Fujwara, et al, Lancet (1980) 1:55-used a protein-depleted surfactant preparation derived from bovine lungs; the preparation is effective but immunogenic. Hallman, M., et al, Pediatrics (1983) 71:473-482 used a surfactant isolate from human amniotic fluid to treat a limited number of infants with some success. U.S. Patent 4,312,860 to Clements discloses an artificial surfactant which contains no protein and is said to be useful in this approach although no data are shown. In short, surfactant substitution has not been widely used clinically.

The preferred surfactant substitute would be the lung surfactant complex itself. This complex is composed of apoprotein, two phospholipids (dipalmitoyl phosphocholine (DPPC) and phosphatidyl-glycerol (PG)) which are present in major amount, several lipid components present in only very minor amount, and calcium ions. The apoprotein contains proteins having molecular weights of the order of 32,000 daltons and very hydrophobic proteins of the order of about 10,000 daltons (King, R.J. et al, Am J Physiol (1973) 224:788-795). The 32,000 dalton protein is glycosylated and contains hydroxyproline.

Analysis of human ASP species by gel electrophoresis have been reported in Chemical Abstracts 99 no. 102 (1983), abstract 155807m and Biochimica et Biophysica Acta 791 (1984), 226-238. Canine ASP species have been studied by Sueishi et al, Biochimica et Biophysica Acta 665 (1981), 442-453 and by King et al, Am. J. Physiol. 224, 788-795 (1973).

A major reason for the limited progress in surfactant replacement therapy has been the lack of availability of the protein portion of the complex. Replacement therapies have focused on attempts to use the lipid components alone, and it appears that the performance of such treatment can be markedly improved by addition of the apoprotein (Hallman, M., et al, Pediatric Clinics of North America (1982) (supra)). At present, however, these proteins are available only from normal adult human lung, and from amniotic fluid. Even efficient isolation procedures would not provide an adequate supply. Thus, it would be desirable to have available a method for producing practical quantities of apoprotein for use alone or in conjunction with the saturated phospholipid portion of the complex.

### Disclosure of Invention

The invention provides a means for obtaining the apoprotein portion of the lung surfactant complex in quantity and under conditions which permit optimization of its features. The remaining components of the complex, dipalmitoyl phosphocholine and phosphatidylglycerol, along with calcium ions are already readily available. The availability of required quantities of manipulable apoprotein both makes possible research efforts to optimize the form of complex useable in therapy, and opens the possibility for routine replacement therapy of respiratory distress syndrome.

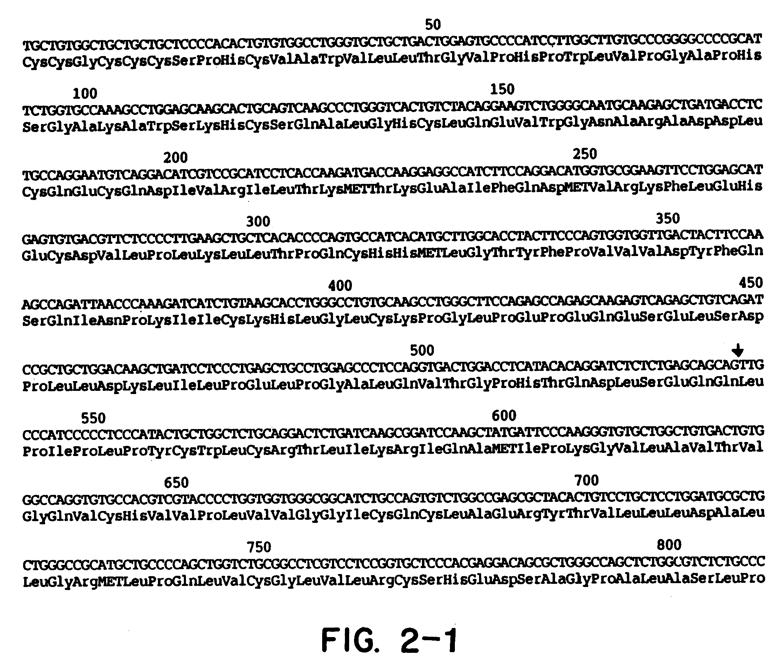

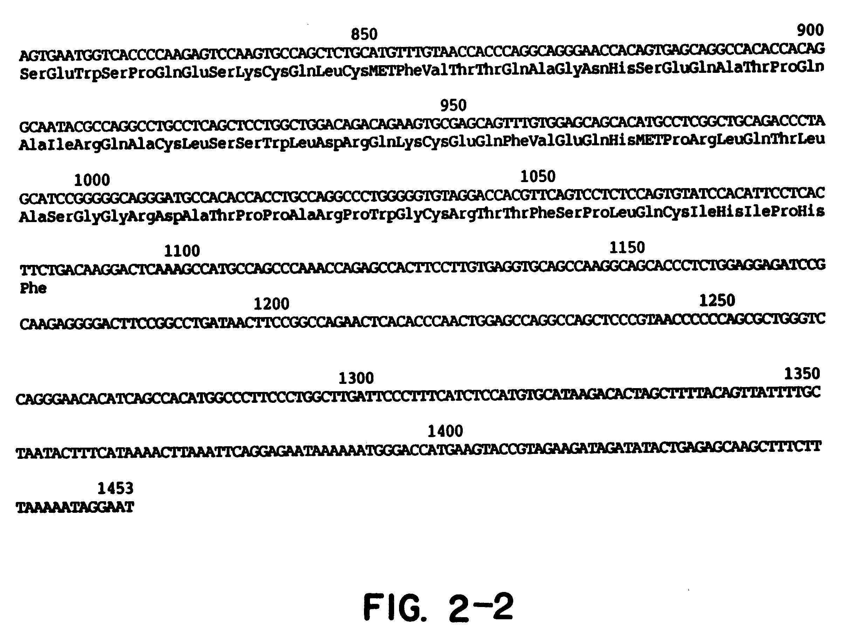

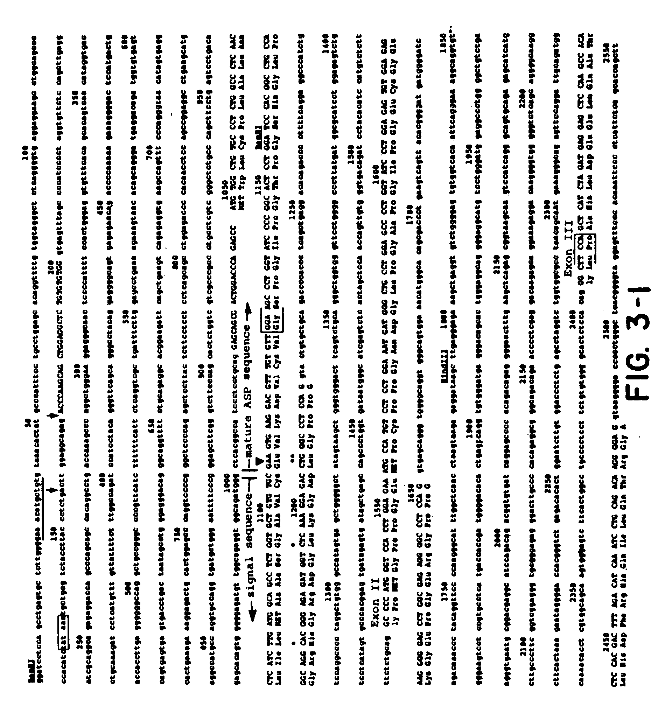

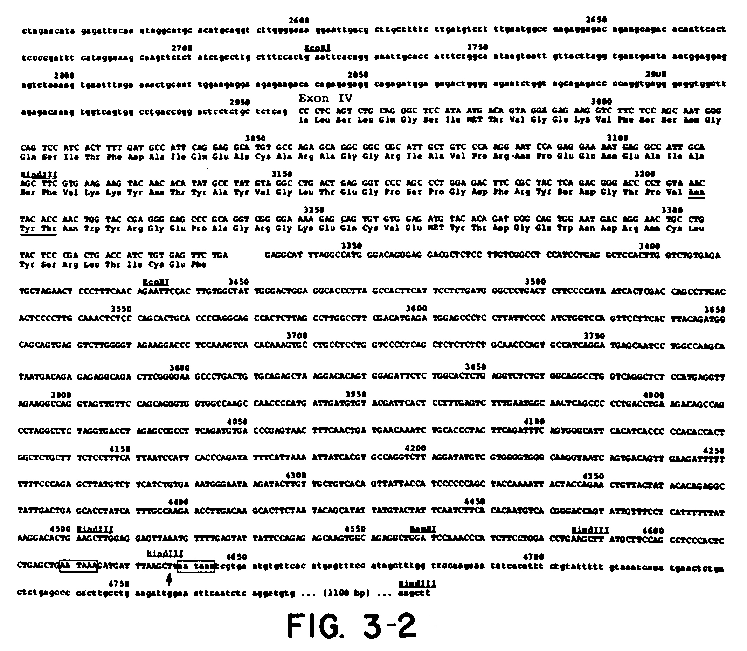

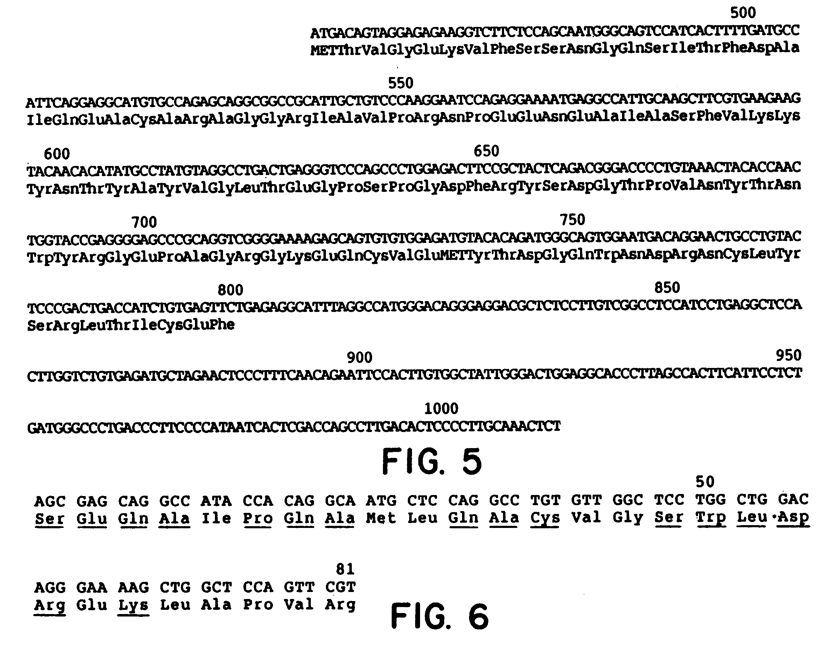

Thus, in one aspect, the invention relates to a mammalian alveolar surfactant protein (ASP) in purified and isolated form which is:
32 K ASP isolatable from human lung having the encoded amino acid sequence shown in Figure 3 herein;
an ASP isolatable from canine lung exhibiting an apparent molecular weight of about 16.5kd when determined on a non-reducing polyacrylamide gel and about 10-12kd when determined on a reducing polyacrylamide gel and having an amino acid sequence shown as the mature sequence in Figure 2 herein; or
an ASP isolatable from human lung exhibiting an apparent molecular weight of about 16.5kd when determined on a non-reducing polyacrylamide gel and about 10-11kd when determined on a reducing polyacrylamide gel and comprising the amino acid sequence shown in Figure 6 herein.

These proteins encourage formation of surface tension lowering films when complexed with phospholipid in the presence of calcium ion. The invention further relates to DNA sequences encoding the mammalian ASP of the invention, to recombinant expression systems suitable for production of these proteins, to recombinant host cells transformed with these systems, and to methods of producing the recombinant ASPs. In other aspects the invention relates to pharmaceutical compositions containing the ASP of the invention and to such ASP for use in treating RDS.

Hereinafter, the 16.5kd component of the 10-12 or 10-11 kd ASP may be referred to as 10K ASP.

### Brief Description of the Drawings

Figure 1 shows the DNA sequence encoding canine 32K ASP, along with the deduced amino acid sequence.

Figure 2 shows DNA sequence determined for a cDNA encoding canine 10K ASP along with the deduced amino acid sequence.

Figure 3 shows the nucleotide sequence of the human 32K ASP gene and the deduced amino acid sequence.

Figure 4 is an autoradiograph of ³⁵S-Met-labeled, secreted proteins from CHO cells transfected with λ:gHS-15.

Figure 5 shows the sequence of the 3' terminal portion of human ASP cDNA contained in pHS-6.

Figure 6 shows DNA sequence determined for a cDNA encoding human 10K ASP along with the deduced amino acid sequence.

Figure 7 shows the relevant junction and coding sequences of the expression vector pASPc-SV(10).

Figure 8 shows the relevant junction and coding sequences of the expression vector pASPcg-SV(10).

Figure 9 shows the relevant junction and coding sequences of the expression vector pMT-Apo:gHS(HinfI/EcoRI).

Figure 10 shows the results of SDS PAGE conducted on ³⁵S labeled supernatant proteins, with and without treatment with endo F, secreted by pMT-Apo:gHS(HinfI/EcoRI) transfected CHO cells.

Figure 11 shows the results of SDS PAGE immunoblotted with labeled human antiASP conducted on supernatant proteins, with and without treatment with endo F, secreted by pMT-Apo:gHS(HinfI/EcoRI) transfected CHO cells.

Figure 12 shows the results of in vitro determination of the ability of ASP to enhance surface tension lowering by phospholipids.

### Modes of Carrying Out the Invention

### A. Definitions

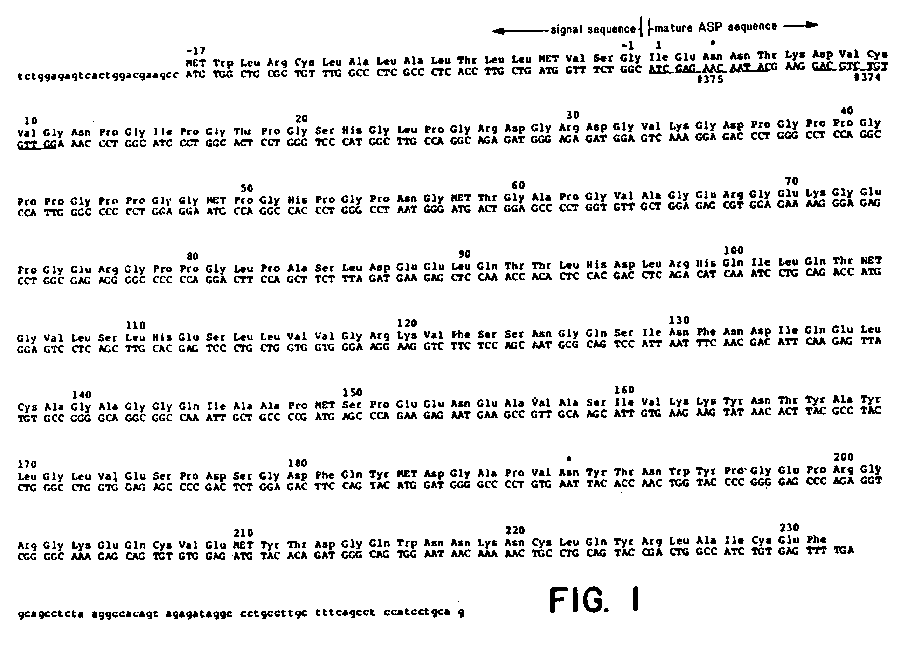

As used herein, "alveolar surfactant protein (ASP)" refers to apoprotein associated with the lung surfactant complex and having ASP activity as defined hereinbelow. The ASP of all species examined appears to comprise one or more components of relatively high molecular weight (of the order of 32 kd) designated herein "32K ASP" and one or more quite hydrophobic components of relatively low molecular weight (of the order of 10-20 kd) designated herein "10K ASP". (King, R.J., et al, J Appl Physiol (1977) 42:483-491; Phizackerley, P.J.R., Biochem J (1979) 183:731-736.) These terms refer to the native sequences and to equivalent modifications thereof. For example, human 32K ASP has the amino acid sequence shown in Figure 3; ASP proteins of approximately 32 kd derived from other species such as dogs, monkeys, or other mammals have substantial degrees of homology with this sequence (see Figure 1 in connection with the canine ASP). Additional sequence for other specific 32K ASP (canine) and 10K ASP (human and canine) is disclosed hereinbelow.

The recombinant ASP proteins of the invention have amino acid sequences corresponding to those of the native proteins.

As is the case for all proteins, the ASP proteins can occur in neutral form or in the form of basic or acid addition salts depending on its mode of preparation, or, if in solution, upon its environment. It is well understood that proteins in general, and, therefore, any ASP, in particular, may be found in the form of its acid addition salts involving the free amino groups, or basic salts formed with free carboxyls. Pharmaceutically acceptable salts may, indeed, enhance the functionality of the protein. Suitable pharmaceutically acceptable acid addition salts include those formed from inorganic acids such as, for example, hydrochloric or sulfuric acids, or from organic acids such as acetic or glycolic acid. Pharmaceutically acceptable bases include the alkali hyroxides such as potassium or sodium hydroxides, or such organic bases as piperidine, glucosamine, trimethylamine, choline, or caffeine. In addition, the protein may be modified by combination with other biological materials such as lipids and saccharides, or by side chain modification, such as acetylation of amino groups, phosphorylation of hydroxyl side chains, or oxidation of sulfhydryl groups or other modification of the encoded primary sequence. Indeed, in its native form, the 32K ASP is a glycosylated protein, and certain of the encoded proline residues have been converted to hydroxyproline. It is also found in association with the phospholipds DPPC and PG. Included within the definition of any ASP herein are glycosylated and unglycosylated forms, hydroxylated and non-hydroxylated forms, the apoprotein alone, or in association with lipids, and, in short, any composition of an amino acid sequence substantially similar to that of the native sequences which retains its ability to facilitate the exchange of gases between the blood and lung air spaces and to permit re-inflation of the alveoli.

It is further understood that minor modifications of primary amino acid sequence may result in proteins which have substantially equivalent or enhanced activity as compared to the native sequences. These modifications may be deliberate, as through site-directed mutagenesis, or may be accidental, such as through mutation of hosts which are ASP producing organisms. All of these modifications are included as long as the ASP activity is retained.

"ASP activity" for a protein is defined as the ability, when combined with lipids either alone or in combination with other proteins, to exhibit activity in the in vivo assay of Robertson, B. Lung (1980) 158:57-68. In this assay, the sample to be assessed is administered through an endotrachial tube to fetal rabbits or lambs delivered prematurely by Caesarian section. (These "preemies" lack their own ASP, and are supported on a ventilator.) Measurements of lung compliance, blood gases and ventilator pressure provide indices of activity. Preliminary assessment of activity may also be made by an in vitro assay, for example that of King, R. J., et al, Am J Physiol (1972) 223:715-726, or that illustrated below of Hawgood, et al, which utilizes a straightforward measurement of surface tension at a air-water interface when the protein is mixed with a phospholipid vesicle preparation.

"Operably linked" refers to a juxtaposition wherein the components are configured so as to perform their usual function. Thus, control sequences operably linked to coding sequences are capable of effecting the expression of the coding sequence.

"Control sequence" refers to a DNA sequence or sequences which are capable, when properly ligated to a desired coding sequence, of effecting its expression in hosts compatible with such sequences. Such control sequences include promoters in both procaryotic and eucaryotic hosts, and in procaryotic organisms also include ribosome binding site sequences, and, in eucaryotes, termination signals. Additional factors necessary or helpful in effecting expression may subsequently be identified. As used herein, "control sequences" simply refers to whatever DNA sequence may be required to effect expression in the particular host used.

"Cells" or "recombinant host cells" or "host cells" are often used interchangably as will be clear from the context. These terms include the immediate subject cell, and, of course, the progeny thereof. It is understood that not all progeny are exactly identical to the parental cell, due to chance mutations or differences in environment. However, such altered progeny are included when the above terms are used.

### B. General Description

The methods illustrated below to obtain DNA sequences encoding ASP are merely for purposes of illustration and are typical of those that might be used. However, other procedures may also be employed, as is understood in the art.

### B.1. The Nature of the Surfactant Complex

The alveolar surface of lung has been studied extensively by a number of techniques, and by a number of groups. It appears that the basement membrane of the alveolus is composed of type I and type II alveolar cells, of which the type II cells comprise approximately 3% of the surface. The type II cells are responsible for the exocrine secretion of materials into a lining fluid layer covering the basement membrane, which materials decrease the surface tension between the liquid of the lining and the gas phase of the contained volume. The fluid layer, then, is comprised of water derived from the blood plasma of the alveolar capillaries, and the surfactant secretions of the type II cells.

The type II cells, themselves, contain 60-100 pg of protein and about 1 pg of lipid phosphorus per cell where the ratio between type II cell DPPC and PG phosphorus is about 8 to 1. Studies of the apoprotein components have been based on pulmonary lavage from various species, and have been shown to comprise two major proteins, as discussed above, of approximate molecular weights 10-20 kd and of 32 kd (Kikkawa, Y., et al, Laboratory Investigation (1983) 49:122-139.) It is not clear whether the apoproteins are bound to the phospholipid component (King, R. J., et al, Am Rev Respir Dis (1974) 110:273) or are not (Shelly, S. A., et al, J Lipid Res (1975) 16:224).

It has been shown that the higher molecular weight protein obtained by pulmonary lavage of dogs, and separated by gel electrophoresis is composed of 3 major components of molecular weight 29,000, 32,000, and 36,000 daltons. The 32,000 dalton protein was used to obtain sequence data, as set forth below; however, all 3 of these proteins have identical N-terminal sequences, and there is evidence that they differ only in degree of glycosylation. Digestion of the 36 kd and 32 kd bands with endoglycosidase F, which removes carbohydrate side chains, results in products which co-migrate with the 29 kd component. The mobility of the 29 kd component is unaffected by this treatment. It has also been shown that the 32 kd fraction aggregates into dimers and trimers.

The smaller molecular weight proteins are extracted with more difficulty, but these, too, appear to be mixtures (Phizackerley, et al (supra): description below).

### B.2. Cloning of Coding Sequences for Canine and Human ASP Proteins

The entire canine and human ASP 32K protein encoding sequences have been cloned, and are available for expression in a variety of host cells as set forth in ¶C below. In addition, DNA sequences encoding several of the lower molecular weight proteins from both human and canine sources have also been obtained.

The canine 32K sequence was obtained from a cDNA library prepared from mRNA isolated from adult canine lung, by probing with two sets of synthetic oligonucleotides, one prepared to accomodate all the possible sequences encoding amino acids 1-5 of the N-terminal sequence and the other amino acids 7-11 of that sequence, as well as a single 15-mer encoding the amino acids 1-5, selected on the basis of mammalian codon preference. Immobilized cDNA from the library constructed in E. coli was probed using these oligonucleotide sets. False positives were minimized by requiring hybridization to more than one set. Successfully hybridizing clones were sequenced, and one was shown to contain the correct N-terminal sequence.

The cDNA insert from the successful clone, excised with PstI, was then used as a probe of the original canine cDNA library, to obtain two additional clones containing inserts encoding other regions of the ASP which, together with this probe, span 844 nucleotides containing the complete coding sequence of canine 32K ASP. The entire nucleotide sequence of the three appropriate inserts, and the deduced 256 amino acid sequence are shown in Figure 1.

This same originally retrieved N-terminal encoding fragment used above was also used as a probe to obtain fragments from a human genomic library in λ phage Charon 28. The entire coding sequence for human ASP 32K protein was found to be contained in a single phage plaque, and to be contained within 2 contiguous BamHI fragments, a 5' 1.2 kb and a 3' 3.5 kb fragment. The pertinent portions of these fragments, encoding human ASP, and containing 3 introns, are shown in Figure 3; the deduced amino acid sequence of human ASP, contains 228 amino acids, and is preceded by a signal sequence of at least 25 amino acids. The human 32K ASP cDNA corresponding to the full length protein was also obtained by probing human cDNA libraries derived from human fetal and adult lung mRNA.

Extensive homology exists between the canine and human 32K amino acid sequences.

Similar strategies were followed in obtaining cDNA encoding human and canine 10K ASP. The canine lung cDNA library described above was probed with two synthetic oligomer mixtures designed to correspond to the N-terminal amino acid sequence of a 16.5 kd (on unreduced gels) canine protein, and clones hybridizing to both probes were recovered and sequenced. One of these clones, which contained canine ASP encoding sequence, was used to probe a cDNA library prepared in bacteriophage gt10 from mRNA isolated from adult human lung to obtain a human 10K ASP encoding clone.

### B.3. Expression of ASP

As the nucleotide sequences encoding the various human and canine ASP are now available, these may be expressed in a variety of systems as set forth in ¶C. If procaryotic systems are used, an intronless coding sequence should be used, along with suitable control sequences. The cDNA clones for any of the above ASP proteins may be excised with suitable restriction enzymes and ligated into procaryotic vectors for such expression. For procaryotic expression of ASP genomic DNA, the DNA should be modified to remove the introns, either by site-directed mutagenesis, or by retrieving corresponding portions of cDNA and substituting them for the intron-containing genomic sequences. The intronless coding DNA is then ligated into expression vectors for procaryotic expression.

As exemplified below, ASP encoding sequences may also be used directly in an expression system capable of processing the introns, usually a mammalian host cell culture. To effect such expression, the genomic sequences can be ligated downstream from a controllable mammalian promoter which regulates the expression of these sequences in CHO cells.

### B.4. Protein Recovery

The ASP protein may be produced either as a mature protein or a fusion protein, or may be produced along with a signal sequence in cells capable of processing this sequence for secretion. It is advantageous to obtain secretion of the protein, as this minimizes the difficulties in purification; thus it is preferred to express the human ASP gene which includes the codons for native signal sequence in cells capable of appropriate processing. It has been shown that cultured mammalian cells are able to cleave and process heterologous mammalian proteins containing signal sequences, and to secrete them into the medium (McCormick, F., et al, Mol Cell Biol (1984) 4:166).

When secreted into the medium, the ASP protein is recovered using standard protein purification techniques. The purification process is simplified, because relatively few proteins are secreted into the medium, and the majority of the secreted protein will, therefore, already be ASP. However, while the procedures are more laborious, it is within the means known in the art to purify this protein from sonicates or lysates of cells in which it is produced intracellularly in fused or mature form.

### B.5. Assay for ASP Activity

In vitro methods have been devised to assess the ability of ASP proteins to function by reducing surface tension (synonymous with increasing surface pressure) to generate a film on an aqueous/air interface. Studies using these methods have been performed on the isolated native 10K canine ASP. (Benson, B.J., et al Prog Resp Res (1984) 18:83-92: Hagwood, S., et al, Biochemistry (1985) 24:184-190.)

Tanaka, Y. et al, Chem Pharm Bull (1983) 31:4100-4109 disclose that a 35 kd protein obtained from bovine lung enhanced the surface spreading of DPPC; Suzuki, Y., J Lipid Res (1982) 23:62-69; Suzuki, Y., et al. Prog Resp Res (1984) 18:93-100 showed that a 15 kd protein from pig lung enhanced the surface spreading of the lipid-protein complex from the same source.

Since the function of the surfactant complex in vivo is to create a film at the air/aqueous interface in order to reduce surface tension, the ability of ASP proteins to enhance the formation of the film created by the spread of lipid or lipoprotein at such a surface in an in vitro model is clearly relevant to its utility.

### B.6. Administration and Use

The purified proteins can be used alone and in combination in pharmaceutical compositions appropriate for administration for the treatment of respiratory distress syndrome in infants or adults. The compositions and protein products of the invention are also useful in treating related respiratory diseases such as pneumonia and bronchitis. For use in such treatment, either of the components, but preferably the 32K component, either alone or, even more preferably, in combination with the 10K component of human ASP is combined with natural or synthetic lipids to reconstruct a surfactant complex. The complex contains about 50% to almost 100% (wt/wt) lipid and 50% to less than 1% ASP; preferably ASP is 5%-20% of the complex. The lipid portion is preferably 80%-90% (wt/wt) DPPC with the remainder unsaturated phosphatidyl choline, phosphatidyl glycerol, triacylglycerols, palmitic acid or mixtures thereof. The complex is reassembled by mixing a solution of ASP with a suspension of lipid lipsomes, or by mixing the lipid protein solutions directly in the presence of detergent or an organic solvent. The detergent or solvent may then be removed by dialysis.

While it is possible to utilize the natural lipid component from lung lavage in reconstructing the complex, and to supplement it with appropriate amounts of ASP proteins, the use of synthetic lipids is clearly preferred. First, there is the matter of adequate supply, which is self-evident. Second, purity of preparation and freedom from contamination by foreign proteins, including infectious proteins, which may reside in the lungs from which the natural lipids are isolated, are assured only in the synthetic preparations. Of course, reconstitution of an effective complex is more difficult when synthetic components are used.

While the 32K human ASP may be used alone as the protein component of the compositions, the combination of 32K and 10K proteins is preferred. The protein ratio is typically in the range of 80:20 for 32K:10K group. The 32K protein may be added directly to an aqueous suspension of phospholipid vesicles in an aqueous solution; because it is so hydrophobic, the 10K protein is added to the lipids in an organic solvent, such as chloroform, the solvents evaporated, and the vessicles re-formed by hydration.

The compositions containing the complex are preferably those suitable for endotracheal administration, i.e., generally as a liquid suspension, as a dry powder "dust" or as an aerosol. For direct endotracheal administration, the complex is suspended in a liquid with suitable excipients such as, for example, water, saline, dextrose, or glycerol and the like. The compositions may also contain small amounts of non-toxic auxiliary substances such as pH buffering agents, for example, sodium acetate or phosphate. To prepare the "dust", the complex, optionally admixed as above, is lyophylized, and recovered as a dry powder.

If to be used in aerosol administration, the complex is supplied in finely divided form along with an additional surfactant and propellent. Typical surfactants which may be administered are fatty acids and esters, however, it is preferred, in the present case, to utilize the other components of the surfactant complex, DPPC and PG. Useful propellents are typically gases at ambient conditions, and are condensed under pressure. Lower alkanes and fluorinated alkanes, such as Freon, may be used. The aerosol is packaged in a container equipped with a suitable valve so that the ingredients may be maintained under pressure until released.

The surfactant complex is administered, as appropriate to the dosage form, by endotracheal tube, by aerosol administration, or by nebulization of the suspension or dust into the inspired gas. Amounts of complex between about 0.1 mg and 10 mg are administered in one dose. For use in newly born infants, one administration is generally sufficient. For adults, sufficient reconstituted complex is administered to replace demonstrated levels of deficiency (Hallman, M., et al, J Clinical Investigation (1982) 70:673-682).

### C. Standard Methods

Most of the techniques which are used to transform cells, construct vectors, extract messenger RNA, prepare cDNA libraries, and the like are widely practiced in the art, and most practitioners are familiar with the standard resource materials which describe specific conditions and procedures. However, for convenience, the following paragraphs may serve as a guideline.

### C.1. Hosts and Control Sequences

Both procaryotic and eucaryotic systems may be used to express the ASP encoding sequences; procaryotic hosts are the most convenient for cloning procedures. Procaryotes most frequently are represented by various strains of E. coli; however, other microbial strains may also be used. Plasmid vectors which contain replication sites and control sequences derived from a species compatible with the host are used; for example, E. coli is typically transformed using derivatives of pBR322, a plasmid derived from an E. coli species by Bolivar, et al, Gene (1977) 2:95. pBR322 contains genes for ampicillin and tetracycline resistance, and thus provides additional markers which can be either retained or destroyed in constructing the desired vector. Commonly used procaryotic control sequences which are defined herein to include promoters for transcription initiation, optionally with an operator, along with ribosome binding site sequences, include such commonly used promoters as the beta-lactamase (penicillinase) and lactose (lac) promoter systems (Chang, et al, Nature (1977) 198:1056 and the tryptophan (trp) promoter system (Goeddel, et al Nucleic Acids Res (1980) 8:4057 and the lambda derived P_{L} promoter and N-gene ribosome binding site (Shimatake, et al, Nature (1981) 292:128).

In addition to bacteria, eucaryotic microbes, such as yeast, may also be used as hosts. Laboratory strains of Saccharomyces cerevisiae, Baker's yeast, are most used although a number of other strains are commonly available. Vectors employing, for example, the 2 µ origin of replication of Broach, J. R., Meth Enz (1983) 101:307, or other yeast compatible origins of replications (see, for example, Stinchcomb, et al, Nature (1979) 282:39, Tschempe, et al, Gene (1980) 10:157 and Clarke, L, et al, Meth Enz (1983) 101:300) may be used. Control sequences for yeast vectors include promoters for the synthesis of glycolytic enzymes (Hess, et al, J Adv Enzyme Reg (1968) 7:149; Holland, et al, Biochemistry (1978) 17:4900). Additional promoters known in the art include the promoter for 3-phosphoglycerate kinase (Hitzeman, et al, J Biol Chem (1980) 255:2073), and those for other glycolytic enzymes. Other promoters, which have the additional advantage of transcription controlled by growth conditions are the promoter regions for alcohol dehydrogenase 2, isocytochrome C, acid phosphatase, degradative enzymes associated with nitrogen metabolism, and enzymes responsible for maltose and galactose utilization. It is also believed terminator sequences are desirable at the 3' end of the conding sequences. Such terminators are found in the 3' untranslated region following the coding sequences in yeast-derived genes.

It is also, of course, possible to express genes encoding polypeptides in eucaryotic host cell cultures derived from multicellular organisms. See, for example, Tissue Cultures, Academic Press, Cruz and Patterson, editors (1973). These systems have the additional advantage of the ability to splice out introns and thus can be used directly to express genomic fragments. Useful host cell lines include VERO and HeLa cells, and Chinese hamster ovary (CHO) cells. Expression vectors for such cells ordinarily include promoters and control sequences compatible with mammalian cells such as, for example, the commonly used early and late promoters from Simian Virus 40 (SV 40) (Fiers, et al, Nature (1978) 273:113), or other viral promoters such as those derived from polyoma, Adenovirus 2, bovine papiloma virus, or avian sarcoma viruses. The controllable promoter, hMTII (Karin, M., et al, Nature (1982) 299:797-802) may also be used. General aspects of mammalian cell host system transformations have been described by Axel; U.S. Patent No. 4,399,216 issued 16 August 1983. It now appears, also that "enhancer" regions are important in optimizing expression; these are, generally, sequences found upstream or downstream of the promoter region in non-coding DNA regions. Origins of replication may be obtained, if needed, from viral sources. However, integration into the chromosome is a common mechanism for DNA replication in eucaryotes.

### C.2. Transformations

Depending on the host cell used, transformation is done using standard techniques appropriate to such cells. The calcium treatment employing calcium chloride, as described by Cohen, S. N., Proc Natl Acad Sci (USA) (1972) 69:2110, or the RbCl₂ method described in Maniatis, et al, Molecular Cloning: A Laboratory Manual (1982) Cold Spring Harbor Press, p. 254 may be used for procaryotes or other cells which contain substantial cell wall barriers. For mammalian cells without such cell walls, the calcium phosphate precipitation method of Graham and van der Eb, Virology (1978) 52:546, optionally as modified by Wigler, M., et al, Cell (1979) 16:777-785 may be used. Transformations into yeast may be carried out according to the method of Van Solingen, P., et al, J Bact (1977) 130:946 or of Hsiao, C. L., et al, Proc Natl Acad Sci (USA) (1979) 76:3829.

### C.3. Probing cDNA or Genomic Libraries

cDNA or genomic libraries are screened using the colony hybridization procedure. Each microtiter plate is replicated onto duplicate nitrocellulose filter papers (S & S type BA-85) and colonies are allowed to grow at 37°C for 14-16 hr on L agar containing 15 µg/ml tetracycline. The colonies are lysed with 10% SDS and the DNA is fixed to the filter by sequential treatment for 5 min with 500 mM NaOH/1.5 M NaCl, then 0.5 M Tris HCl(pH 8.0)/1.5 M NaCl followed by 2 x standard saline citrate (SSC). Filters are air dried and baked at 80°C for 2 hr.

For nick-translated probe, the duplicate filters are prehybridized at 42°C for 16-18 hr with 10 ml per filter of DNA hybridization buffer (50% formamide (40% formamide if reduced stringency), 5 x SSC, pH 7.0, 5x Denhardt's solution (polyvinylpyrrolidine, plus Ficoll and bovine serum albumin; 1 x = 0.02% of each), 50 mM sodium phosphate buffer at pH 7.0, 0.2% SDS, 50 µg/ml yeast tRNA, and 50 µg/ml denatured and sheared salmon sperm DNA).

Samples are hybridized with nick-translated DNA probes at 42°C for 12-36 hr for homologous species and 37°C for heterologous species contained in 5 ml of this same DNA hybridization buffer. The filters are washed two times for 30 min, each time at 50°C, in 0.2 x SSC, 0.1% SDS for homologous species hybridization, and at 50°C in 3 x SSC, 0.1% SDS for heterologous species hybridization Filters are air dried and autoradiographed for 1-3 days at -70°C.

For synthetic (15-30 mer) oligonucleotide probes, the duplicate filters are prehybridized at 42°C for 2-8 hr with 10 ml per filter of oligo-hybridization buffer (6 x SSC, 0.1% SDS, 1 mM EDTA, 5x Denhardt's, 0.05% sodium pyrophosphate and 50 µg/ml denatured and sheared salmon sperm DNA).

The samples are hybridized with kinased oligonucleotide probes of 15-30 nucleotides under conditions which depend on the composition of the oligonucleotide. Typical conditions employ a temperature of 30-42°C for 24-36 hr with 5 ml/filter of this same oligo-hybridization buffer containing probe. The filters are washed two times for 15 min at 23°C, each time with 6 x SSC, 0.1% SDS and 50 mM sodium phosphate buffer at pH 7, then are washed once for 2 min at the calculated hybridization temperature with 6 x SSC and 0.1% SDS, air dried, and are autoradiographed at -70°C for 2 to 3 days.

### C.4. cDNA Library Production

Double-stranded cDNA is synthesized and prepared for insertion into the plasmid vector pBR322 using homopolymeric tailing mediated by calf thymus terminal transferase (Sutcliffe, J. G., Nucleic Acid Res (1978) 5:2721-2732). First strand cDNA is synthesized by the RNA-dependent DNA polymerase from Avian Myeloblastosis Virus, by priming with oligo (dT) 12-18 on 5 µg mRNA. The RNA template is then liberated from the nascent DNA strand by denaturation at 100°C for 5 min, followed by chilling on ice. Second strand DNA is synthesized by using the large fragment of DNA polymerase I of E. coli, relying on self-priming at the 3'-end of the first strand molecule, thereby forming a double-stranded hairpin DNA. These molecules are blunt-ended at the open-ended termini, and the hairpin loop is cleaved open with S1 nuclease from Aspergillus oryzae. S1 nuclease digestion of the double-stranded cDNA takes place in 300 mM NaCl, 30 mM NaOAc, pH 4.5, 3 mM ZnCl₂ for 30 min at 37°C with 600 units enzyme. The cDNA is extracted with phenol:chloroform, and small oligonucleotides are removed by three ethanol precipitations in the presence of ammonium acetate. This is done as follows: a half volume of 7.5 M ammonium acetate and two volumes ethanol are added to the cDNA solution, which is precipitated at -70°C. The blunt-ended, double-stranded cDNA is then fractionated by size using gel filtration through a column (0.3 x 14 cm) Sepharose 4B (Pharmacia Fine Chemicals, Piscataway, NJ) or by ultracentrifugation in 5-20% glycerol gradient followed by fractionation of the gradient. cDNA roughly greater than the desired length, e.g., 300 base pairs is retained and recovered by precipitation with 70% ethanol. Short (10-30 nucleotides) polymeric tails of deoxycytosine are added to the 3' termini of the cDNA using a reaction containing 0.2 M potassium cacodylate, 25 mM Tris, pH 6.9, 2 mM dithiothreitol, 0.5 mM CoCl₂, 200 mM cDTP, 400 µg/ml BSA, and 40 units calf thymus terminal deoxynucleotide transferase for 5 min at 22°C. The reaction is extracted with phenol:chloroform, and small oligonucleotides are removed with three ethanol precipitations in the presence of ammonium acetate.

The dC-tailed cDNA is annealed with pBR322 which has been cleaved with PstI and tailed with oligo dG: 2.5 µg pBR322-dG DNA is annealed with the cDNA at a vector concentration of 5 µg/ml, and the hybrids are transferred into E. coli MC1061 by the CaCl₂-treatment described by Casabadan, M., et al, Mol Biol (1980) 138:179-207.

### C.5. Vector Construction

Construction of suitable vectors containing the desired coding and control sequences employs standard ligation and restriction techniques which are well understood in the art. Isolated plasmids, DNA sequences, or synthesized oligonucleotides are cleaved, tailored, and religated in the form desired.

Site specific DNA cleavage is performed by treating with the suitable restriction enzyme (or enzymes) under conditions which are generally understood in the art, and the particulars of which are specified by the manufacturer of these commercially available restriction enzymes. See, e.g., New England Biolabs, Product Catalog. In general, about 1 µg of plasmid or DNA sequence is cleaved by one unit of enzyme in about 20 µl of buffer solution; in the examples herein, typically, an excess of restriction enzyme is used to insure complete digestion of the DNA substrate. Incubation times of about one hour to two hours at about 37°C are workable, although variations can be tolerated. After each incubation, protein is removed by extraction with phenol/chloroform, and may be followed by ether extraction, and the nucleic acid recovered from aqueous fractions by precipitation with ethanol. If desired, size separation of the cleaved fragments may be performed by polyacylamide gel or agarose gel electrophoresis using standard techniques. A general description of size separations is found in Methods in Enzymology (1980) 65:499-560.

Restriction cleaved fragments may be blunt ended by treating with the large fragment of E. coli DNA polymerase I (Klenow) in the presence of the four deoxynucleotide triphosphates (dNTPs) using incubation times of about 15 to 25 min at 20 to 25°C in 50 mM Tris pH 7.6, 50 mM NaCl, 6 mM MgCl₂, 6 mM DTT and 5-10 µM dNTPs. The Klenow fragment fills in at 5' sticky ends but chews back protruding 3' single strands, even though the four dNTPs are present. If desired, selective repair can be performed by supplying only one of the, or selected, dNTPs within the limitations dictated by the nature of the sticky ends. After treatment with Klenow, the mixture is extracted with phenol/chloroform and ethanol precipitated. Treatment under appropriate conditions with S1 nuclease or Bal-31 results in hydrolysis of any single-stranded portion.

Synthetic oligonucleotides are prepared by the method of Efimov, V. A., et al (Nucleic Acids Res (1982) 6875-6894), and can be prepared using commercially available automated oligonucleotide synthesizers. Kinasing of single strands prior to annealing or for labeling is achieved using an excess, e.g., approximately 10 units of polynucleotide kinase to 1 nmole substrate in the presence of 50 mM Tris, pH 7.6, 10 mM MgCl₂, 5 mM dithiothreitol, 1-2 mM ATP, 1.7 pmoles γ32P-ATP (2.9 mCi/mmole), 0.1 mM spermidine, 0.1 mM EDTA.

Ligations are performed in 15-50 µl volumes under the following standard conditions and temperatures: 20 mM Tris-Cl pH 7.5, 10 mM MgCl₂, 10 mM DTT, 33 µg/ml BSA, 10 mM-50 mM NaCl, and either 40 µM ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 33-100 µg/ml total DNA concentrations (5-100 nM total end concentration). Intermolecular blunt end ligations (usually employing a 10-30 fold molar excess of linkers) are performed at 1 µM total ends concentration.

In vector construction employing "vector fragments", the vector fragment is commonly treated with bacterial alkaline phosphatase (BAP) or calf intestinal alkaline phosphatase (CIP) in order to remove the 5' phosphate and prevent religation of the vector. Digestions are conducted at pH 8 in approximately 150 mM Tris, in the presence of Na⁺ and Mg⁺² using about 1 unit of BAP or CIP per µg of vector at 60° for about one hour. In order to recover the nucleic acid fragments, the preparation is extracted with phenol/chloroform and ethanol precipitated. Alternatively, religation can be prevented in vectors which have been double digested by additional restriction enzyme digestion of the unwanted fragments.

For portions of vectors derived from cDNA or genomic DNA which require sequence modifications, site specific primer directed mutagenesis is used. This is conducted using a primer synthetic oligonucleotide complementary to a single stranded phage DNA to be mutagenized except for limited mismatching, representing the desired mutation. Briefly, the synthetic oligonucleotide is used as a primer to direct synthesis of a strand complementary to the phage, and the resulting double-stranded DNA is transformed into a phage-supporting host bacterium. Cultures of the transformed bacteria are plated in top agar, permitting plaque formation from single cells which harbor the phage.

Theoretically, 50% of the new plaques will contain the phage having, as a single strand, the mutated form; 50% will have the original sequence. The resulting plaques are hybridized with kinased synthetic primer at a temperature which permits hybridization of an exact match, but at which the mismatches with the original strand are sufficient to prevent hybridization. Plaques which hybridize with the probe are then picked, cultured, and the DNA recovered. Details of site specific mutation procedures are described below in specific examples.

### C.6. Verification of Construction

In the constructions set forth below, correct ligations for plasmid construction are confirmed by first transforming E. coli strain MC1061 obtained from Dr. M. Casadaban (Casadaban, M., et al, J Mol Biol (1980) 138:179-207) or other suitable host with the ligation mixture. Successful transformants are selected by ampicillin, tetracycline or other antibiotic resistance or using other markers depending on the mode of plasmid construction, as is understood in the art. Plasmids from the transformants are then prepared according to the method of Clewell, D. B., et al, Proc Natl Acad Sci (USA) (1969) 62:1159, optionally following chloramphenicol amplification (Clewell, D. B., J Bacteriol (1972) 110:667). The isolated DNA is analyzed by restriction and/or sequenced by the dideoxy method of Sanger, F., et al, Proc Natl Acad Sci (USA) (1977) 74:5463 as further described by Messing, et al, Nucleic Acids Res (1981) 9:309, or by the method of Maxam, et al, Methods in Enzymology (1980) 65:499.

### C.7. Hosts Exemplified

Host strains used in cloning and expression herein are as follows:
For cloning and sequencing, and for expression of construction under control of most bacterial promoters, E. coli strain MC1061 was used.

For M13 phage recombinants, E. coli strains susceptible to phage infection, such as E. coli strain JM101 are employed.

The cells used for expression are Chinese hamster ovary (CHO) cells.

### D. Cloning and Expression of ASP

Both canine and human ASP proteins were obtained in purified form. Canine cDNA was used to provide probes for the human ASP genomic and cDNA library.

### D.1. Purification of Canine ASP

### D.1.a. Isolation of the Surfactant Complex

Lung surfactant complex was prepared from canine lungs obtained from exsanguinated canines. All procedures, including the lavage, were performed at 4°C and the isolated material was stored at -15°C.

The lungs were degassed and lavaged 3 times with one liter per lavage of 5 mM Tris-HCl, 100 mM NaCl, pH 7.4 buffer. The Ca⁺² concentration of this buffer was less than 5 x 10⁻⁶ M (Radiometer F2112 Ca; Radiometer A/S, Copenhagen, Denmark). The pooled lung washings were spun at 150 x gₐᵥ for 15 min (Sorval RC2-B) to remove cellular material. The supernatant was then spun at 20,000 x gₐᵥ for 15 hr (Beckman L3-40) using a type 15 rotor (Beckman Instruments), and the resulting pellet was dispersed in buffer containing 1.64 M sodium bromide. After equilibration for 1 hr, the suspension was spun at 100,000 x gₐᵥ for 4 hr (Beckman L5-50B) in a SW28 rotor (Beckman Instruments). The pellicle was resuspended in buffer and spun at 100,000 x gₐᵥ for 1 hr (Beckman L5-50B). This pellet containing the complex was resuspended in double distilled water.

### D.1.b. Extraction of Lipid and 10 kd Protein

Pellet resuspended in water at a concentration of 10-15 mg phospholipid/ml was injected into a 50-fold volume excess of n-butanol (Sigrist, H., et al, Biochem Biophys Res Commun (1977) 74:178-184) and was stirred at room temperature for 1 hr. After centrifugation at 10,000 x gₐᵥ for 20 min (Sorval RC2-B), the pellet, which contains the 32K ASP is recovered for further purification as described below. The supernatant, which is a single phase, contains the lipids and the lower molecular weight proteins. To obtain the lipids, the supernatant was dried under vacuum at 40°C and the lipids were extracted (Folch, J., et al, J Biol Chem (1957) 226:497-509).

To obtain the hydrophobic protein, the supernatant was subjected to rotovap to remove the butanol, and further dried by addition of ethanol followed by rotovap. The dried residue was suspended in redistilled chloroform containing 0.1 N HCl, and insoluble material removed by centrifugation.

The resulting solution was chromatographed over an LH-20 column (Pharmacia) and developed in chloroform. (LH-20 is the hydroxypropyl derivative of Sephadex G-50; it is a hydrophobic gel which is inert to organic solvents.) The proteins are excluded; lipids/phospholipids elute from the included volume.

Protein is recovered from the void volume fractions by evaporation of the chloroform under nitrogen, and then subjected to sizing on polyacrylamide gels. When run under non-reducing conditions, three bands of 16.5 kd, 12 kd, and 10 kd were obtained; under reducing conditions, a single broad band of 10-12 kd was found.

The 16.5 kd and 12 kd bands from the non-reduced gels were subjected to N-terminal analysis by Edman degradation, to give the following sequences:

For 16.5 kd:   ?-Pro-Ile-Pro-Leu-Pro-Tyr-Cys-Trp-Leu-Cys-
Arg-Thr-Leu-Ile-Lys-Arg-Ile-Gly-Ala-Met-Ile-
Pro-Lys-Gly-Val-Leu-Ala-Val-Thr- ? -Gly-Gln-

For 12 kd:   Ile-Pro-Cys-Phe-Pro-Ser-Ser-Leu-Lys-Arg-Leu-
Leu-Ile-Ile-Val-Trp

### D.1.c. Protein Fractionation and Verification as ASP 32K Protein

The precipitate from the n-butanol extraction above was dried under nitrogen and washed twice in 20 ml of buffer containing 20 mM octyl-β-D-glucopyranoside. After centrifugation at 100,000 x gₐᵥ for 1 hr (Beckman L5-50B), the pellet was dispersed in 0.3 M lithium diiodosalicylate, 0.05 M, pyridine (pH 8.4) on ice, diluted with an equal volume of water, and mixed with a volume of n-butanol equal to the aqueous phase. A total of 9 n-butanol-water partitions were performed to lower the detergent concentration in the aqueous phase. The final lower, aqueous phase containing the protein was lyophilized for 15 hr, taken up in 2 ml of buffer and spun at 100,000 x gₐᵥ (Beckman L5-50B) to remove any remaining insoluble material. The lithium diiodosalicylate concentration in the final sample, calculated from an extinction coefficient of 4 x 10³ at 323 nm (Marchesi, V. T. and Andrews, E. P., Science (1971) 174:1247-1248), was less than 10 µM.

The thus purified canine ASP 32K apoprotein was reconstituted with surfactant lipids purified as above. The reconstituted material had surface activity as measured by the surface balance and its in vivo biological activity was demonstrated by inspiration into fetal rabbits maintained on a ventilator.

### D.1.d. Further Protein Purification

The protein fraction obtained in the previous subparagraph was reduced by incubation with 50 mM DTT in 1% SDS, 50 mM Tris-HCl, 1 mM EDTA pH 7.5 at 37°C for 1 hr, aklylated with 100 mM iodoacetamide (Sigma) at 0°C for 30 min, and subjected to polyacrylamide gel eletrophoresis by the procedure of Laemmli, U. K., Nature (1970) 227:680-685. The proteins were visualized by soaking the gel in 4 M sodium acetate solution and the 32K band was sliced out with a razor blade, and electroluted by the protocol of Hunkapiller, M. W., et al, Methods in Enzymology (1983) 91:227-235, New York, Academic Press, using the CBS Scientific (Del Mar, California) electrolution device.

The eluted protein was lyophilized and its N-terminal amino acid sequence was determined from one nanomole of protein using the Applied Biosystems 470A gas-phase sequencer (Applied Biosystems Inc., Foster City, CA) in accordance with the instructions of the manufacturer. PTH amino acids were identified with a Beckman 334T HPLC, using a 0.46 x 25 cm IBM CN-column. The gradient applied was as indicated in Hunkapiller, N. W., and Hood, L. E., Methods in Enzymology (1983) 91:486-492, New York, Academic Press, with the following modifications: Instead of a binary gradient system a ternary gradient system was used in which acetonitrile and methanol were pumped by separate pumps and the ratio of the two varied with time over the course of the gradient, with appropriate modification of the gradient program; instead of the Permaphase ETH^{r} guard column, a "5 x 0.46 cm IBM CN" analytical "mini-column", was used; and the column was heated to 28°C, rather than to 32°C.

The N-terminal amino acid sequence was:
1                    5                        10
Ile-Glu-Asn-Asn-Thr-Lys-Asp-Val-Cys-Val-Gly-Asn-
15                       20                       25
Hyp-Gly-Ile-Hyp-Gly-Thr-Hyp-Gly-Ser-His-Gly-Leu-Hyp-Gly-
30
Arg-?-Gly-Arg-?-Gly-Val.

"Hyp" indicates the modified amino acid hydroxyproline.

Amino acid composition data for the canine 32K protein show a hydroxyproline content consistent with the hydroxylation of proline residues in the deduced sequence (see below) which appear in the collagen-like pattern Gly-X-Hyp. As this pattern is also shown in the human N-terminal sequence it is probable, by analogy to the canine data, that similarly disposed prolines in the human sequence are hydroxylated.

Information regarding processing was obtained by purification and sequencing of collagenase treated canine ASP.

Purified canine ASP was digested with bacterial collagenase (Worthington, Freehold NJ) at a 1:1 enzyme:substrate ratio in 5 mM Tris pH 7.4-5 mM CaCl₂ at 37°C. That produced a 22 kd limit digest product as analyzed on SDS gels. This 22 kd band was electroeluted from a gel and subjected to amino acid sequence analysis as described above. Two amino acids were identified at each cycle, indicating the the collagenase treatment had produced two peptides which remain linked by a disulfide bridge. From the cDNA clone sequence it can be demonstrated that the two sequences correspond to amino acids 78-110 and 203-231 in the intact molecule. The sequences obtained are: 
2 4 6 8 10 12 14
Gly-Pro-Hyp-Gly-Leu-Pro-Ala-Ser-Leu-Asp-Glu-Glu-Leu-Gln-
Gly-Lys-Glu-Gln-Cys-Val-Glu-Met-Tyr-Thr-Asp-Gly-Gln-Trp-

16 18 20 22 24 26 28
Thr-Thr-Leu-His-Asp-Leu-Arg-His-Gln-Ile-Leu-Gln-Thr-Met-
Asn-Asn-Lys-Asn-Cys-Leu-Gln-Tyr-Arg-Leu-Ala-Ile-Cys-Glu-

30 32
Gly-Val-Leu-Ser-Leu
Phe
and demonstrate that translation is complete, and that the C-terminus of the protein is intact.

### D.1.e. Isolation of Human ASP

Human 32K and lower molecular weight ASP was prepared following the procedure described above for canine proteins from a patient suffering from alveolar proteinosis (a syndrome which results from the presence of excess surfactant in the lung).

The 32K ASP has the N-terminal sequence: 
1                    5                        10
Glu-Val-Lys-Asp-Val-Cys-Val-Gly-Ser-Hyp-Gly-Ile-
          15
Hyp-Gly-Thr-Hyp-Gly
Amino acids 3-17 of the human sequence are precisely homologous to amino acids 6-20 of the canine 32K protein except for the serine at position 9.

The isolated low molecular weight hydrophobic proteins show bands corresponding to 16.5 kd, 14 kd and 10 kd when subjected to polyacrylamide gel electrophoresis under non-reducing conditions. Under reducing conditions, a single broad band corresponding to 10-11 kd is obtained.

### D.2. Isolation of Canine Lung mRNA

Total RNA was isolated from an adult canine lung by the method of Chirgwin, J. M., et al, Biochemistry (1979) 18:5294-5299. The lung tissue was first pulverized by grinding with a mortar and pestle in liquid N₂, and homogenized in a solution of 6 M guanidine thiocyanate, 0.05 M Tris-HCl, pH 7.0, 0.1 M-β-mercaptoethanol, 0.5% Sarcrosyl. This homogenate was made 2.0 M in CsCl and layered over a 5.7 M CsCl cushion in 0.01 M ethylenediaminetetraacetic acid (EDTA) and 0.05 M Tris-HCl, pH 9.0. The RNA was pelleted through this cushion by centrifugation at 115,000 x g for 16 hr, thereby separating it from the cellular DNA and protein which do not sediment through the higher density CsCl solution. The RNA was then dissolved in 0.01 M Tris-HCl, pH 7.4, 0.005 M EDTA, 1.0% sodium dodecylsulfate (SDS), extracted with a 1:1 mixture of chloroform and phenol, and precipitated from 70% ethanol. The polyadenylated RNA (poly A⁺ RNA) fraction was obtained by affinity chromatography through oligo (dT) cellulose as described by Aviv, H., and Leder, P., Proc Natl Acad Sci (USA) (1972) 69:1840-1412.

### D.3. Construction and Screening of Canine Lung cDNA Library

Adult canine lung poly A⁺ RNA prepared as in ¶D.2 was used to construct a cDNA library as described in ¶C.4, 5 µg mRNA yielded about 25 ng of cDNA, size-selected to greater than 300 base pairs. The library contained about 200,000 independent recombinants. Of these, 40,000 recombinants were plated on nitrocellulose filters. These filters served as the masters for subsequent replicas (in accordance with the method of Hanahan, D., and Meselson, M., Gene (1980) 10:63-75.

### cDNA Encoding the 32K Protein

Three probes were constructed: a mixture of 24 x 14-mer sequences complementary to the amino acids 1-5 having the sequence
(probe a); 64 x 14-mers complementary to the amino acids 7-11 having the sequence 
            A           A
5' GACGTTTGCGTTGG 3'
     T      C    T      C
            G           G
(probe b); and a single 15-mer
5' ATCGAGAACAACACC 3'
selected on the basis of mammalian codon preference (probe c). Each oligonucleotide mixture and the single unique oligonucleotide were synthesized on a Biosearch SAM I oligonucleotide synthesizer (Biosearch, Inc., San Rafael, CA) by a modification of the standard phosphotriester method using mesitylenesulfonyl chloride in the presence of N-methylimidazole as condensing reagents as described by Efimov, V. A., et al, Nucleic Acids Res (1982) 10:6875-6894, and purified by polyacrylamide gel electrophoresis.

For hybridization, xix replica filters were prepared from each master filter, so that each colony could be screened in duplicate with each of three oligonucleotide probes. Colonies recovered after replication off the master filters were placed on agar plates containing 170 µg/ml chloramphenicol for 18 hr. The colonies were then prepared for hybridization according to the method of Grunstein, M., and Hogness, D., Proc Natl Acad Sci (1975) 72:3961-3972.

The filters were baked for 2 hr at 80°C under vacuum and then washed for 4 hr at 68°C with shaking in a large volume of 3X SSC (where 1X SSC is 0.15 M NaCl, 0.015 M sodium citrate, pH 7.5), 0.1% SDS. The filters were prehybridized in 6 x SSC, 0.1% SDS, 1 mM EDTA, 5x Denhardt's solution (0.1% Ficoll, 0.1% polyvinylpyrrolidone, 0.1% bovine serum albumin) 0.05% sodium pyrophosphate and 50 µg/ml denatured salmon sperm DNA at 42°C for a minimum of 2 hr.

Duplicate filters were then hybridized with 5 x 10⁶ cpm of one of each ³²P-labeled oligonucleotide probe (phosphoryltated in accordance with Maniatis, T., et al, Molecular Cloning, (1982) Cold Spring Harbor Laboratories, pp. 122-123) per filter in 10 ml hybridization solution containing identical ingredients as the prehybridization solution. Filters with oligonucleotide probes a, b, and c were hybridized at 37°C, 45°C, and 41°C, respectively. After 1 hr, the thermostat was lowered to 28°C for probe a and 37°C for probe b, after which the bath was allowed to equilibrate. Filters with probe c were not hybridized at a lower temperature. The filters were washed twice in 6 x SSC, 0.1% SDS at room temperature for 15 min, then washed in 6 x SSC, 0.1% SDS at 37°C, 45°C, and 41°C for probes a, b, and c, respectively, for 2 min. The final washing temperature was obtained form the empirical formula of Suggs, S. V., et al, Developmental Biology Using Purified Genes (ed. D. D. Brown and C. F. Fox), Academic Press, NY, pp. 683-693; that is, ${\text{T}}_{\text{d}} \text{= 4(G+C) + 2(A+T)}$ . The hybridized filters were then dried and autoradiographed on Kodakâ XAR film with Dupontâ Cronex intensifying screens until complete exposures were obtained.

A colony was considered positive if it hybridized in duplicate with all three oligonucleotide probes or with both probes a and b. Of several potential positive clones, one hybridized much more intensely with probes a and b as compared to the others. Sequencing of this clone demonstrated that it encoded a portion of the sequence of canine 32K ASP. It was designated DS-1 and used to obtain the entire 32K canine ASP.

The purified DNA insert of 375 base pairs was excised from pDS-1 by restriction with PstI and prepared using small miniprep methods (Maniatis, et al, supra at p. 366) and was isolated on agarose gels. The intact DNA insert was then subcloned into bacteriophage M13 (Messing, J., and Vieira, J., Gene (1982) 19:259-268) and sequenced using the dideoxy method of Sanger, F., et al, Proc Natl Acad Sci (USA) (1977) 74:5463-5469. The sequence encoded the N-terminal portion of the approximately 300 amino acid protein, i.e., the 32 residue N-terminal amino acid sequence determined from the purified canine ASP of ¶D.1, and 101 additional downstream amino acids. It also contained 50 base pairs of the 5' untranslated region.

The mRNA pool was assessed to determine the presence of sequences of sufficient length to encode the entire canine ASP sequence by Northern blot. Poly A⁺ RNA of ¶D.2 was subjected to Northern blot using nick translated DS-1 insert DNA after fractionation by electrophoresis on a 1.4% agarose gel containing methylmercuric hydroxide by the method of Bailey, J. M. and Davidson, N., Anal Biochem (1976) 70:75-85. mRNA hybridizing to probe was 1800-2000 nucleotides in length, clearly larger than the approximately 700 nucleotides needed for the coding sequence.

The DS-1 insert probe was therefore used to rescreen one duplicate set of original filters, which had been treated at 100°C for 10 min to remove residual oligonucleotide probe. Filters were prehybridized in 0.75 M NaCl, 0.075 M Na citrate, 50% formamide, 0.5% SDS, 0.02% bovine serum albumin, 0.02% Ficoll - 400,000, 0.02% polyvinyl pyrollidone, 0.1% sodium pyrophosphate, 50 µg.ml yeast tRNA and 50 µg/ml denatured sheared salmon sperm DNA) at 42°C for 18 hr. 5 x 10⁵ cpm of ³²P-labeled boiled DS-1 cDNA was added per ml fresh hybridization buffer and the filters were incubated in this buffer at 42°C for 16 hr. They were then washed in 0.03 M NaCl and 0.003 M sodium citrate and 0.1% SDS two times each for 30 min at 50°C, and exposed for autoradiography overnight. Two additional clones, DS-4 and DS-31, were identified, which, together with DS-1, comprise roughly 1700 base pairs (Figure 1).

DS-4 and DS-31 were also excised using PstI, subcloned into the PstI site of M13mp9, and sequenced by dideoxy sequencing according to the procedure of Sanger, F. (supra). The entire sequence contains two internal PstI sites. Confirmation of correct sequencing was obtained by dideoxy sequencing of fragments obtained from deduced internal restriction sites, as shown in Figure 1. The entire nucleotide sequence including the amino acid sequence of ASP deduced from the 256 codon open reading frame is shown in Figure 1.

### cDNA Encoding Canine 10K ASP

Two oligomeric probes were synthesized corresponding to the N-terminal sequence of the 16.5 kd protein using mammalian codon preference tables for codon choice. Probe 1198 was a 36-mer of the sequence 5'-GGTCACAGCCAGGCCCTTGGGGATCATGGCCTGGAT-3'; probe 1199 was a 45-mer of the sequence 5'-CTTGATCAGGGTTCTGCACAGCCAGCAGTAGGGCAGGGGGATGGG-3'. Both were labelled with ³²P by kinasing.

For hybridization, filters were baked at 80°C for two hours under vacuum and then washed for 4 hr at 68°C with shaking in a large volume of 3 x SSC containing 0.1% SDS. The filters were prehybridized for several hours at 42°C in 6 x SSc, 5 x Denhardt's, 20% formamide, 0.1% SDS, and 100 µg/ml sheared, denatured salmon sperm DNA. Duplicate filters were hybridized in the above buffer containing either 13 ng/ml probe 1198 or 16 ng/ml probe 1199 at an intial temperature of 68°C, and then at 42°C overnight. The filters were washed twice for 15 min at room temperature in 6 x SSC, 0.1% SDS, 0.05% sodium pyrophosphate, then for 5 min at 65°C in the same buffer, and then dried and autoradiographed.

Of 40,000 clones screened, 8 hybridized to both probes, and were subjected to restriction analysis. Two overlapping clones which when combined span 1520 nucleotides were sequenced, with the results shown in Figure 2. The arrow indicates the beginning of the mature 16.5 kd protein.

### D.4. Isolation of the human 32K ASP Gene

A human genomic library cloned into bacteriophage Charon 28 (Rimm, D. L., et al, Gene (1980) 12:301-310) was obtained from Dr. T. Maniatis, Harvard University. Approximately 1.5 x 10⁶ phage were grown on E. coli K803, and plaque lysates were transferred to nitrocellulose filters as described by Benton, W. D., et al, Science (1977) 196:180-182. The filters were probed with DS-1 cDNA which had been kinased by the nick-translation method of Rigby, P. W. J., et al, J Mol Biol (1977) 113:237-251. Filters were prewashed in hybridization buffer (0.75 M NaCl, 0.75 M sodium nitrate, 40% formamide, 0.05% SDS, 0.02% bovine serum albumin, 0.02% Ficoll - 400,000, 0.02% polyvinyl pyrollidone, 0.1% sodium pyrophosphate, 50 µg/ml yeast tRNA, 50 µg/ml denatured sheared salmon sperm DNA) at 42°C for 1 hr. 5 x 10⁵ cpm probe was added per ml fresh hybridization buffer and the filters were incubated in this buffer at 37°C for 16 hr. They were then washed in 0.45 M NaCl and 0.045 M sodium citrate and 0.1% SDS two times at 50°C, and exposed for autoradiography overnight. Six potential clones containing sequences hybridizing to DS-1 cDNA were purified. The most strongly hybridizing clone, gHS-15, was characterized.

A 700 bp EcoRI fragment from gHS-15 hybridized with the DS-1 probe and was chosen for sequence analysis. This EcoRI fragment was purified, inserted into M13mp9, sequenced and found to be extensively homologous with the corresponding canine sequence.

The entire human coding region was contained within two contiguous BamHI fragments: a 5' 1.2 kb and a 3' 3.5 kb fragment. Both BamHI fragments were individually subcloned into the BamHI site of M13mp8 and sequenced. Additional fragments were similarly sequenced according to the strategy shown in Figure 3. The sequence information was analyzed using various Intelligenetics (Palo Alto, CA) computer programs in accordance with the instructions of the manufacturer. The regions containing the signal peptide, precursor sequence and mature apoprotein were identified by comparison to the canine ASP cDNA. From the sequence analysis, the 5' terminus of the gene is encoded within the 1.2 kb BamHI fragment and the 3' terminus within the 3.5 kb BamHI fragment. The gene is interrupted by three introns at positions 1218 bp, 1651 bp and 2482 bp, with position 1 being the first bp of the 1.2 kb BamHI fragment. The entire sequence, including the amino acid sequence of human ASP protein deduced is shown in Figure 3.

### D.5. Expression of Human 32K ASP

The phage isolate gHS-15 identified in ¶D.5 as harboring an insert of approximately 16 kb containing the entire human ASP gene was transferred into CHO cells which had been grown in McCoy's 5A medium with 10% fetal bovine serum by co-transformation with pSV2:NEO (Southern, P., et al, J Mol Appl Genet (1982) 1:327-341), a plasmid containing a functional gene conferring resistance to the neomycin analog G148, which is toxic to mammalian cells. In the transformation, 15 µg of the λ:gHS-15 and 2 µg of pSV2:NEO were applied to a 100 mm dish of CHO cells in a calcium phosphate/DNA coprecipitate according to the method of Wigler, M., et al, Cell (1979) 16:777-785, with inclusion of a 2 min "shock" with 15% glycerol 4 hr after exposure to the DNA. The cells were transferred to medium containing 1 µg/ml G418, and yielded about 50 stable transformants per 100 mm dish.

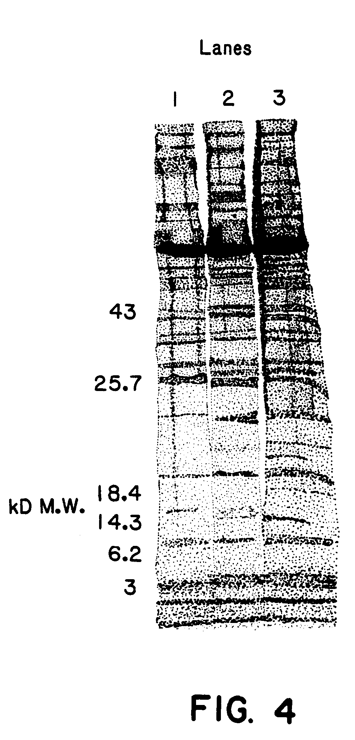

Stable transformants were cultured prior to labeling in media supplemented with 0.25 mM ascorbic acid. Two pools of stable transformants and one pool of untreated CHO cells were grown for 1 hr in medium containing 1/10 of normal methionine concentration and then labeled with ³⁵S-methionine for 8-16 hours, and the ³⁵S-met labeled total secreted proteins were analyzed by SDS-polyacrylamide gel electrophoresis. The results are shown in Figure 4. Lane 1 shows the normal CHO secreted proteins. Lanes 2 and 3 display λ:gHS-15 secreted proteins: both of which have an additional 30-36 kd protein corresponding to an expressed ASP protein. To further document the identity of the 30-36 kd protein one can immunoprecipitate the total secreted protein samples with canine ASP antibodies. The vector λ:gHS-15 was deposited with the American Type Culture Collection on 7 December 1984 and has accession no. ATCC 40146.

### D.6. Preparation of a Human cDNA Clones for the 32K and 10K Proteins

### Human 32K ASP

Human lung was obtained from two fetuses, one 22 weeks, the other 24 weeks of age. 7 g of lung tissue was first pulverized by grinding with a mortar and pestel in liquid N₂, and total poly A⁺ RNA prepared as set forth in ¶D.2 (supra).

A cDNA library was prepared from the mRNA as set forth in ¶C.4. Five µg of lung poly A⁺ RNA yielded about 25 ng of cDNA, size-selected to greater than 500 base pairs, and gave a library of 300,000 independent recombinants.

60,000 members of the human cDNA library were screened with the canine DS-1 cDNA in the manner described above for the screening of the genomic library. The recombinant colonies were plated on nitrocellulose filters which served as masters for two sets of replicas. The colony filters were then prepared for hybridization according to the method of Grunstein, M., and Hogness, D. (supra). The filters were baked for 2 hr at 80°C under vacuum and then washed for 4 hr at 68°C with shaking in a large volume of 3X SSC and 0.1% SDS. Next the filters were prehybridized in 0.75 M NaCl, 0.075 M sodium nitrate, 40% formamide, 0.5% SDS, 0.02% bovine serum albumin, 0.02% Ficoll - 400,000, 0.02% polyvinyl pyrollidone, 50 µg/ml yeast tRNA, 50 µg/ml denatured sheared salmon sperm DNA) at 37°C for 18 hr. One x 10⁶ cpm of ³²P-labeled DS-1 probe was added per ml of fresh hybridization buffer then incubated for 16 hr at 37°C. The filters were then washed in 0.45 M NaCl and 0.045 M sodium citrate and 0.01% SDS two times each for 30 min at 50°C, and exposed for autoradiography overnight.

One positively hybridizing clone, HS-6, was further analyzed by sequence determination; HS-6 harbors a 1.2 kb insert which can be released from the vector using PstI digestion, and which bears an internal EcoRI site. Both PstI-EcoRI fragments from the insert were subcloned into the PstI-EcoRI site of M13mp8 and mp9, and partial sequences obtained. The over 200 bp sequenced portion corresponds perfectly to the 3' end of gHS-15. The nucleotide sequence of HS-6 is shown in Figure 5.

As the HS-6 cDNA insert contained only the 3'-terminal region of the ASP mRNA, the remaining clones were screened for adjacent surfactant sequences using HS-6 as probe. No clones were found in the remainder of the library.

To obtain the complete cDNA encoding human 32K ASP, a randomly primed cDNA was prepared from adult human lung and cloned in the bacteriophage vector gt10 using EcoRI linkers by the procedure of Huynh, T., et al, cDNA Cloning Techniques: A Practical Approach (Glover, D., ed) IRL, Oxford. Adult lung is greatly enriched in ASP transcripts as compared to fetal lung tissue (our observations) and therefore affords a greater frequency of obtaining a complete ASP cDNA.

Phage plaques were screened with a ³²P labelled insert from pHS-6 using 5x10⁻⁵ cpm/ml in 50% formamide, 5 x SSC, 0.05% SDS, 5 x Denhardt's, tRNA and salmon sperm DNA at 42°C for 16 hr. The filters were washed twice at 50°C for 30 min each in 0.2 x SSC, 0.1% SDS, dried and autoradiographed.

Two positively hybridizing clones, designated pHS-2 and pHS-5 were isolated. Each contained the entire 32K ASP encoding sequence and most of the 5' untranslated region. Each overlapped with HS-6, which contained most of the 3' untranslated region: the 3' terminus of each clone corresponds to the EcoRI site within the coding region.

### Human 10K ASP

The same cDNA library in lambda gt10 was screened on nitrocellulose filters as above using 1x10⁶cpm of the canine clone pD10k-1 described above in 40% formamide, 5 x SSC, 0.05% SDS, 5 x Denhardt's, 50 µg/ml yeast tRNA and 50 µg/ml salmon sperm DNA for 16 hr at 37°C. The filters were washed twice at 50°C for 30 min in 2 x SSC, 0.1% SDS, dried and autoradiographed. Of 40,000 plaques, two were positive, and one, designated lambda H10k-1 containing a 1.5 kb insert was chosen for sequencing. Preliminary partial sequencing results are shown in Figure 6. The undelined amino acid residues represent homologies with the canine protein.

### D.7. Construction of Expression Vectors

Vectors suitable for expression of the genomic human 32K ASP encoding sequence in mammalian cells, which are capable of processing intron-containing DNA were constructed. Expression is controlled by the metallothionein II (hMTII) control sequences, as described by Karin, M., et al, Nature (1982) 299:797-802.

The host vector, pMT is obtained by ligating the promoter into pUC8 as follows:
Plasmid 84H (Karin, M., et al (supra)) which carries the hMTII gene was digested to completion with BamHI, treated with exonuclease Bal-31 to remove terminal nucleotides, and then digested with HindIII to liberate an 840 bp fragment containing nucleotides -765 to +70 of the hMTII gene (nucleotide +1 is the first nucleotide transcribed). The 840 bp fragment was isolated and ligated with HindIII/HincII digested pUC8 (Vieira, J., et al, Gene (1982) 19:259-268) and the ligation mixture transformed into E. coli MC1061. The correct construction of pMT was confirmed by dideoxy nucleotide sequencing.

In addition, a derivative of the pMT, pMT-Apo, containing C-terminal regulatory signals was also prepared. pMT-Apo harbors a portion of the human liver protein ApoA₁ gene (Shoulders, C. C., et al, Nucleic Acids Res (1983) 11:2827-2837) which contains the 3'-terminal regulatory signals. A PstI/PstI 2.2 kb fragment of ApoA₁ gene (blunt ended) was cloned into the SmaI site of the pMT polylinker region, and the majority of the ApoA₁ gene removed by digestion with BamHI, blunt ending with Klenow, digestion with StuI, and religation. The resulting vector contains roughly 500 bp of the ApoA₁ gene from the 3' terminus as confirmed by dideoxy-sequence analysis.

Five constructs of the human ASP gene and the pMT and pMT-Apo expression vectors were prepared using the 1.2 kb and 3.5 kb BamHI fragments of gHS-15. All constructs were isolated and confirmed by both restriction analysis and dideoxy sequencing. These constructs were prepared as follows:
1. the 1.2 kb and 3.5 kb BamHI fragments were cloned into the BamHI site of pMT to give pMT:gHS;
2. the 1.2 kb BamHI fragment was truncated at the 5' terminus by digestion with HinfI (position 950) and filled in with Klenow. The truncated fragment was cloned, along with the 3.5 kb fragment into the BamHI site of pMT to give pMT:gHS(HinfI);
3. the fragments of ¶2 were cloned instead into the BamHI site of pMT-Apo to give pMT-Apo:gHS(HinfI);
4. the 3.5 kb BamHI fragment was truncated at the 3' terminus by digestion with EcoRI (position 3434) and filled in with Klenow. This truncated fragment was cloned, along with the truncated 1.2 kb fragment truncated with HinfI as above into the BamHI site of pMT-Apo to give pMT-Apo:gHS(HinfI/EcoRI);
5. the 1.2 kb fragment was truncated at the BstEII site at position 356 and the 3.5 kb fragment at the BetEII site at position 4024. These fragments were cloned into the BamHI site of pMT-Apo to give pMT-Apo:gHS(BstEII).

The resulting pMT:gHS constructs were transferred into CHO cells as set forth in ¶D.6 except that 10⁻⁴ M ZnCl₂ was added with ³⁵S-methionine to induce the metallothionein promoter and label the proteins produced.

After 8-16 hr the medium is analyzed for ³⁵S-met labeled total secreted protein which immunoprecipitates with antibodies to canine ASP. Non-immune IgG are used as a control.

### D.8. Optimization of Expression

Conditions of expression were optimized, and additional expression vectors containing the SV40 viral enhancer were used to increase the levels of expression in CHO cells. Three vectors were used: pMT-Apo:gHS(HinfI/EcoRI) described above and further characterized below, and pASPc-SV(10) and pASPcg-SV(10) which are constructed as described below.

### Enhancer-containing Vectors

To obtain host expression vectors containing the SV40 enhancer in operable linkage to the MT-II promoter an 1100 bp SV40 DNA fragment was inserted into the HindIII site preceding the MT-II promoter sequences in pMT. The SV40 DNA fragment spans the SV40 origin of replication and includes nucleotide 5171 through nucleotide 5243 (at the origin), the duplicated 72 bp repeat from nucleotide 107-250, and continues through nucleotide 1046 on the side of the origin containing the 5' end of late viral mRNAs. This HindIII 1100 bp fragment is obtained from a HindIII digest of SV40 DNA (Buchman, A.R., et al, DNA Tumor Viruses, 2d ed (J. Tooze, ed.), Cold Spring Harbor Laboratory, New York (1981), pp. 799-841), and cloned into pBR322 for amplification. The cloning vector was cut with HindIII, and the 1100 bp SV40 DNA fragment isolated by gel electrophoresis and ligated into HindIII-digested, CIP-treated, pMT. The resulting vectors, designated pMT-SV(9) and pMT-SV(10), contain the fragment in opposite orientations preceding the MT-II promoter. In pMT-SV(9), the enhancer is about 1600 bp from the 5' mRNA start site; in the opposite orientation it is approximately 980 bp from the 5' mRNA start site. Both orientations are operable, but the orientation wherein the enhancer sequences are proximal to the start site provides higher levels of expression.

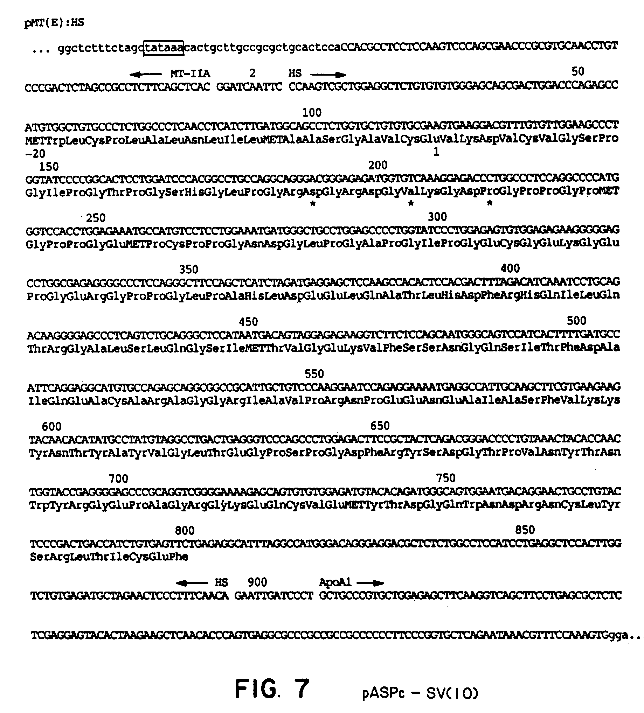

pASPc-SV(10): The coding sequences for ASP were inserted into the above-described modified form of the host vector pMT-SV(10). First, the 500 bp apoAI fragment was inserted into pMt-SV(10) by isolating this fragment, obtained by digestion of pMT-Apo (described above) and ligating the isolate into EcoRI/BamHI digested pMT-SV(10). The modified vector was digested with BamHI, blunted, and ligated to the cDNA sequences obtained from pHS-5 (White, R.T., et al, Nature (1985) 317:361-363) as a blunted EcoRI digest. The cDNA fragment extends from the EcoRI linker joined to the 5' untranslated region to the naturally occurring EcoRI site in the 3' untranslated region (900 bp). The relevant nucleotide sequences are shown in Figure 7, where the starred amino acids represent differences in the primary amino acid sequence from that of the protein obtained from pMT-Apo:gHS(HinfI/EcoRI). (The differences result from base changes between human cDNA and the genomic sequences.) Initiation of translation is at nucleotide 56, as in the native sequence.

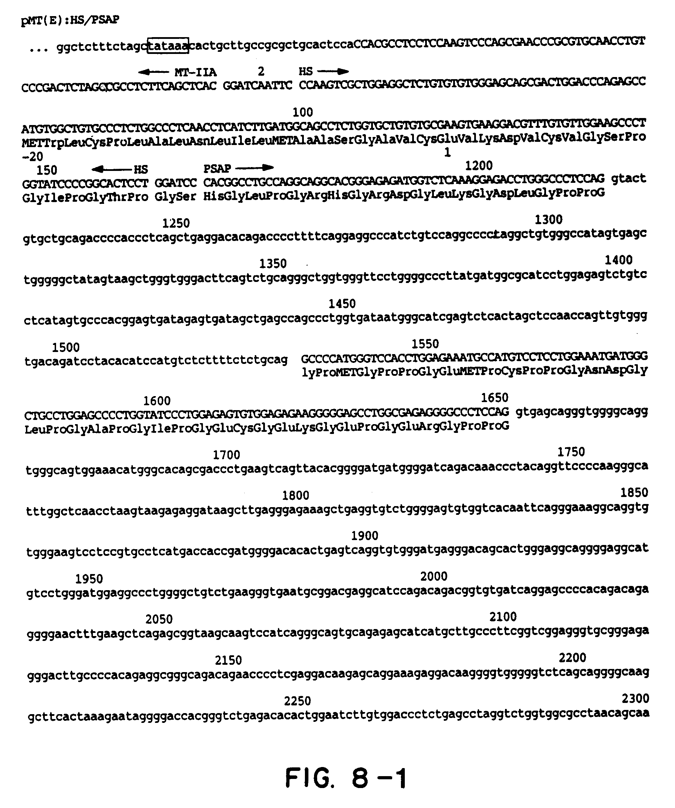

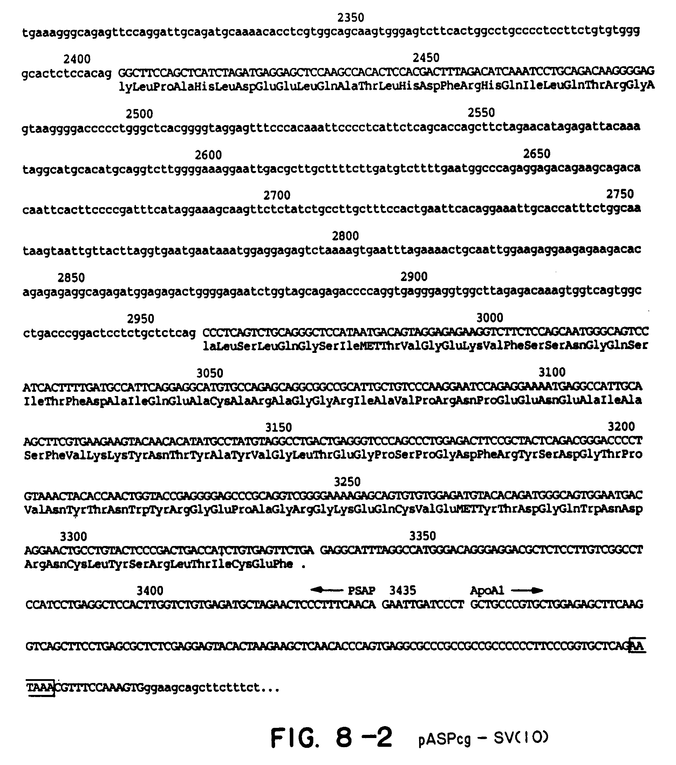

pASPcg-SV(10): An additional modification was prepared by integrating pASPc-SV(10) and pMT-Apo:gHS(HinfI/EcoRI) sequences. Plasmid pASPc-SV(10) was digested with BamHI and EcoRI, and the isolated larger fragment ligated to the 3' portion of the ASP gene obtained by BamHI/EcoRI(partial) digestion of pMT-Apo:gHS(HinfI/EcoRI). This represents the portion of the human ASP gene beginning at nucleotide 1154 and extending to nucleotide 3432, this being ligated to the ApoAI gene fragment as above. This construct results in a protein identical to that obtained from pMT-Apo:gHS(HinfI/EcoRI), but different at amino acid positions 25, 30, and 34 from that obtained from pASPc-SV(10). The nucleotide sequence of the relevant insert is shown in Figure 8.

### PMT-Apo:gHS(HinfI/EcoRI)

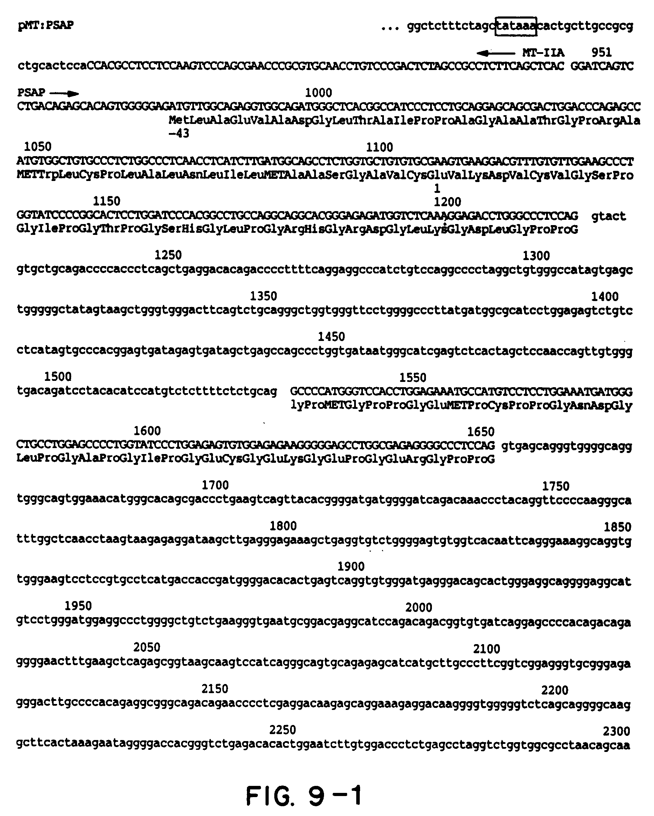

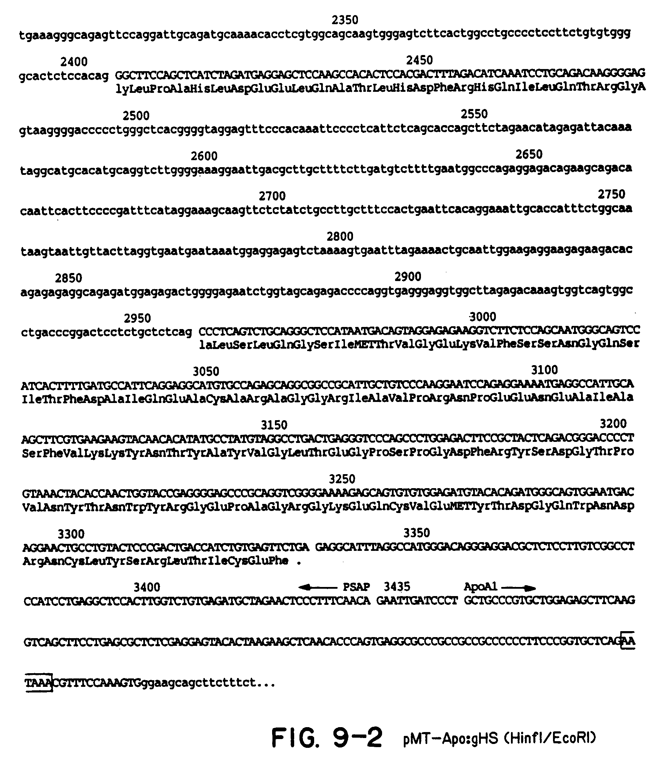

For the genomic DNA-containing vector, pMT-Apo:gHS(HinfI/EcoRI), the coding sequences were obtained as an HinfI/EcoRI fragment of the gene extending from nucleotide 950 to nucleotide 3432, containing exons 2, 3, and 4, and part of exon 5. See also, White, R.T., et al, Nature (1985) 317:361-363. This fragment was ligated to a 500 bp fragment from the 3' end of the human ApoAI gene (Shoulders, C.C., Nucleic Acids Res (1983) 11:2827-2837) which contains the polyadenylation signal and polyadenylation site as set forth above. The entire ASP-encoding genomic insert is shown ligated to the MT-II promoter in Figure 9.

It was expected that this vector would produce a protein 23 amino acids longer than the native preprotein (which includes the signal sequence). The construct lacks exon 1 and therefore translation probably initiates at the ATG beginning at nucleotide 987 of the genomic sequence complementary to native preprotein mRNA, which nucleotide normally resides in the first intron. In the production of native preprotein, exon 1 is spliced to exon 2 at nucleotide 1022, deleting this start codon, and permitting translation to initiate at nucleotide 1046. However, the additional residues do not appear to interfere with secretion, and the normal mature protein is secreted from cells expressing this modified form of the gene.

### Transformation Procedure

Each of the vectors described above was transformed into CHO cells as follows: Chinese hamster ovary (CHO)-K1 cells were grown on medium composed of a 1:1 mixture of Coon's F12 medium and DME21 medium with 10% fetal calf serum. The competent cells were co-transformed with the vector of interest and pSV2:NEO (Southern, P., et al, J Mol Appl Genet (1982) 1:327-341). pSV2:NEO contains a functional gene conferring resistance to the neomycin analog G418. In a typical transformation, 0.5 µg of pSV2-NEO and 5 µg or more of the expression vector DNA are applied to a 100 mm dish of cells. The calcium phosphate-DNA co-precipitation according to the protocol of Wigler, M., et al, Cell (1979) 16:777-785, was used with the inclusion of a two minute "shock" with 15% glycerol in PBS after four hours of exposure to the DNA.

Briefly, the cells are seeded at 1/10 confluence, grown overnight, washed 2x with PBS, and placed in 0.5 ml Hepes-buffered saline containing the CaPO₄·DNA co-precipitate for 15 min and then fed with 10 ml medium. The medium is removed by aspiration and replaced with 15% glycerol in PBS for 1.5-3 min. The shocked cells are washed and fed with culture medium. Until induction of MT-II-controlled expression, the medium contains F12/DMEM21 1:1 with 10% FBS. A day later, the cells are subjected to 1 mg/ml G418 to provide a pool of G418-resistant colonies. Successful transformants, also having a stable inheritance of the desired plasmid, are then plated at low density for purification of clonal isolates.

### Assay for Production Levels of ASP

The transformants are assayed for production of the desired protein, first as pools, and then as isolated clones in multi-well plates. The plate assay levels are somewhat dependent on the well size - e.g. results from 24 well plates are not directly comparable with those from 96 well plates. Clones which are found by plate assay to be producing the protein at a satisfactory level can then be grown in production runs in roller bottles. Typically, the levels of production are higher when the scale up is done. However, there is not an absolute correlation between performance in the plate assay and in roller bottles - i.e. cultures which are the best producers in the plate assay are not necessarily the best after scale-up. For this reason, typically 100-200 or more individual clones are assayed by various screening methods on plates and 5-10 of the highest producers are assayed under production conditions (roller bottle).

### Plate Assays

Pools of cells transformed with the various ASP encoding plasmids were grown in multi-well plates and then exposed to 5 x 10⁻⁵ to 1 x 10⁻⁴ zinc ion concentration to induce production of ASP. ASP assays were conducted using Western blot employing immunoprecipation with rabbit anti-human ASP polyclonal antiserum followed by ¹²⁵I protein A and autoradiography.

In more detail, semiconfluent monolayers of individual cell lines growing in McCoy's 5A medium with 10% FBS were washed with phosphate-buffered saline (PBS) and refed with McCoy's containing 10% FBS, 1 x 10⁻⁴ zinc chloride, and 0.25 mM sodium ascorbate. (Ascorbate may be helpful in mediating the hydroxylation of proline residues.) Twenty-four hours post induction, the cells were washed with PBS and refed with serum-free McCoy's containing the zinc chloride and ascorbate. After 12 hours, the conditioned media were harvested, made 20 mM in Tris, pH 8, and filtered through nitrocellulose in a BRL dot-blot apparatus. The nitrocellulose filter was blocked in 50 mM Tris, pH 7.5, 150 mM NaCl (Tris/salt) containing 5% nonfat dry milk, and then incubated with 1:5000 dilution of rabbit anti-human ASP polyclonal antiserum in the blocking solution, washed several times in the above Tris/salt, and incubated with 25 µCi of ¹²⁵I protein A in blocking solution, washed, and autoradiographed.

Most pools transformed with the ASP encoding vectors did not produce ASP detectable in this assay. However, a positive, ASP-secreting cell line, designated A-38, was selected from pMT-Apo:gHS(HinfI/EcoRI) transformants. In addition, certain pools from cells transformed with pASPc-SV(10), designated ASP-I, or with pASPcg-SV(10), designated ASP-F and ASP-G, produced levels of ASP comparable to those produced by the cell line designated D-4 described below (∼2-5µg/ml).

### Characterization of ASP Protein

The A-38 cells (supra) were grown to 25% confluence in McCoy's 5A medium containing 10% FBS and then induced with 10⁻⁴ M zinc chloride in McCoy's containing 10% FBS and 0.25 mM sodium ascorbate. (Half of the cells were also treated with 10⁻⁶ M dexamethasone.) Twenty-four hours later, the cells were washed with PBS and refed with RPMI medium containing 10% dialyzed FBS, 1 x 10⁻⁴ M zinc chloride, 0.25 mM sodium ascorbate, and 0.5 mCi/ml ³⁵S-methionine.

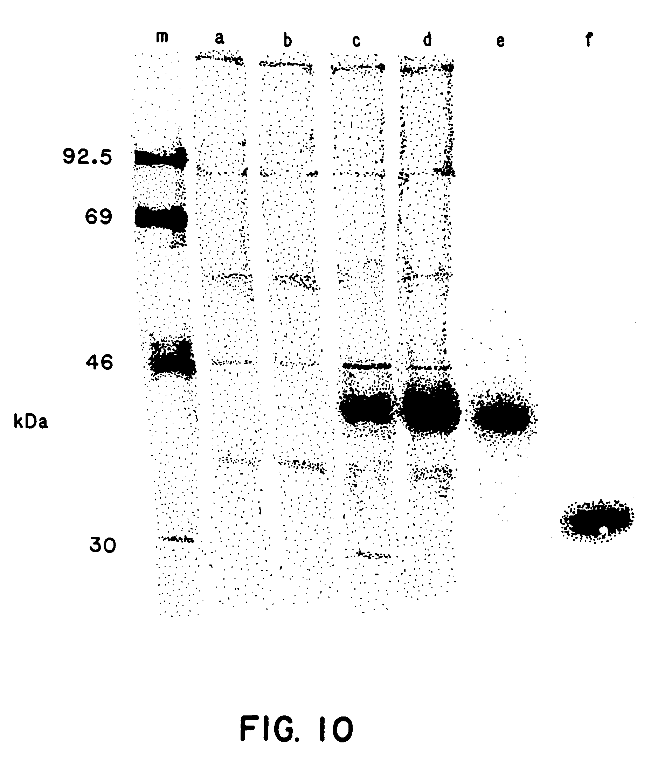

Eighteen hours later, the cell supernatant was made 1 mM phenylmethylsulfonylfluoride and immunoprecipitated with rabbit anti-canine ASP antiserum using protein A as carrier. Half of the precipitated protein was boiled in SDS-PAGE sample buffer, and the other half eluted into 0.75% Triton X-100, 0.075% SDS, 0.75% 2-mercaptoethanol, 30 mM EDTA, 75 mM sodium phosphate, pH 1 and incubated for 1 hr at 37° with 0.5 units of endoglycosidase-F (endo-F). Endo-F treated and untreated protein fractions were subjected to SDS-PAGE with the results shown in Figure 10. The Endo-F treated fraction showed a 30 kd protein (lane F) as compared to 38 kd protein for the untreated (lane E). (Lane M contains size markers, lanes A and B supernatants from untransformed CHO cells, and lanes C and D supernatants from A-38 cells untreated and treated with dexamethasone, respectively.)

### Supertransfection to Prepare D-4

An additional cell line, designated D-4, was obtained by supertransfection of A-38 with a mixture of pMT-Apo:gHS(HinfI/EcoRI) (20 µg) and pSV2:GPT (1 µg). Semiconfluent monolayers of A-38 growing in F12/DMEM21 with 10% FBS were co-transfected, as described above. After 48 hours the cells were split 1:5 into F12/DMEM21 containing 10% FBS and HAT selection drugs. After 17 days of HAT selection, the pool of surviving resistant clones was screened for individual clones producing high levels of ASP by the immunofilter screen method of McCracken, A.A., et al Biotechniques (March/April 1984) 82-87. Briefly, the cells were seeded onto plates at 100 cells per 100 mm dish in F12/DMEM21, 10% FBS. After 5 days (when colonies contain 50-200 cells each), the cells were washed with PBS, refed with serum-free F12/DMEM21, and overlayed with a sterile teflon mesh. On top of the mesh was placed a nitrocellulose filter which was left in place for 8 hr. The nitrocellulose was removed and treated as an immunoblot, first with rabbit anti-canine ASP polyclonal antiserum, then ¹²⁵I protein A, followed by autoradiography. Of approximately 2000 colonies screened, two gave a detectable signal and one, designated D-4, was shown to express the ASP gene at 10-20 times the level of A-38, or at an amount corresponding to an estimated 2-5 µg/ml ASP.

### Characterization

The secreted ASP from the D-4 cell line was isolated from the serum-free medium by affinity chromatography and sequenced at the N-terminus on a gas-phase microsequencer. Determination of a 16 amino acid sequence showed complete homology with the N-terminal portion of the protein isolated from lung lavage; 70% of the total contained an N-terminal Glu residue; the remaining 30% was clipped so as to contain an N-terminal Val (position 2 relative to Glu). This is the same composition as the isolated lavage protein. Hydroxyprolines were present at positions 10, 13, and 16, indicating the ability of the cells to exhibit post-translational processing.

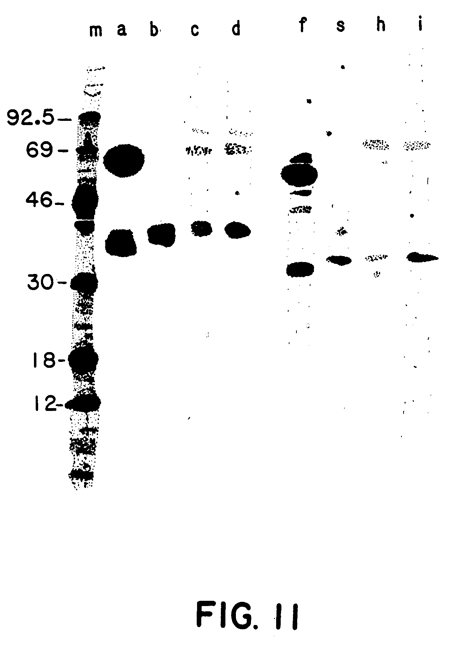

In addition, the protein secreted by D-4 along with the secreted protein fraction from pool ASP-I (supra) and from pool ASP-G (supra) was compared to human proteinosis lung lavage protein using Western blot. Serum-free medium from induced cells was TCA precipitated, treated (or not) with Endo-F and subjected to SDS-PAGE in 12.5% gels. The gel was electroblotted and dot-incubated with rabbit antihuman ASP polyclonal antiserum followed by ¹²⁵I protein A. The results are shown in Figure 11.

Lanes A and F contain 1 µg alveolar proteinosis protein before and after Endo-F digestion: lanes B, C, and D represent media from D-4, ASP-I pool, and ASP-G pool respectively untreated with Endo-F; lanes G, H, and I represent proteins from these supernatants treated with Endo-F. It is evident that Endo-F treatment reduces the apparent molecular weight of all proteins, and results in more discrete bands.

### Production Runs

The supertransfected cell line containing multiple copies of pMT-Apo:gHS(HinfI/EcoRI) (cell line D-4) was used in a production level run in roller bottles. An 850-cm square roller bottle was seeded with a 10 cm dish containing 2 x 10⁶ cells in 10% FCS, 15 mM Hepes, pen/strep, and glutamine. After the cells reached confluence (2-3 days), they were washed 2 x with PBS and replaced with 250 ml of F12/DMEM21, 10 mM Hepes without FCS. The following day the cells were refed with 250 ml of F12/DMEM21, 10 mM Hepes, 5 x 10⁻⁵ zinc chloride, 10⁻⁶ M dexamethasone, and 0.25 mM ascorbate. The cells were harvested every 2 days, spun for ten minutes at 1000 rpm, and frozen at -20°C. Production was 1-5 µg/ml/day, assayed by dot-blot Western using polyclonal anti-canine ASP antisera at 1:5000 dilution, as described above. Production drops after about 14-17 days.

### D.9. Activity of the ASP Components

The ability of the isolated ASP components to enhance the formation of lipid film at an air/aqueous interface was assessed in vitro using the method described by Hagwood, S., et al, Biochemistry (1985) 24:184-190. Briefly, a preparation of phospholipid vesicles with the appropriate ratio of test proteins is added carefully in a small volume to the bottom of a teflon dish containing aqueous buffer, a magnetic stirrer, and a platinum plate suspended at the surface of the buffer and attached to a strain gauge. Changes in surface tension registered on the strain gauge are recorded as a function of time upon starting the stirrer.

10K proteins were added to the phospholipid by mixing a chloroform solution containing them with a 2:1 v/v chloroform:methanol solution of the lipid. The solvents were evaporated, and the solids hydrated in buffer to obtain vesicles. 32K proteins can be added in aqueous solution directly to a suspension of the vesicles, and association with and aggregation of the vesicles can be detected by turbidity measurements.

As reported by Hawgood, et al (supra), 32K canine ASP was capable of aggregating phospholipid vesicles and of enhancing the formation of film when included in the phospholipid vesicles, when the phospholipids were those obtained from the canine lung surfactant complex. The activity of the proteins of the invention is assessed using the same procedures for measuring aggregation and film formation enhancement as set forth in Hawgood.

Both the phospholipid preparation from canine lung prepared as described above (300 µg) and a synthetic mixture of phospholipids were used. The synthetic phospholipid contained 240 µg of commercially available DPPC and 60 µg egg PG, and is much more reluctant to form films than is the natural lipid. However, the test phospholipid was chosen so as to dramatize most effectively the activity of the proteins.

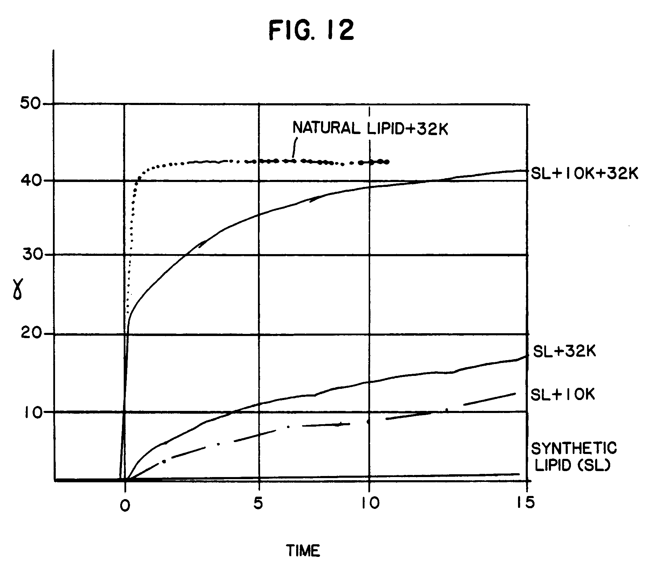

The 32K protein and the mixture of 10K ASP were isolated from canine lung as described above. While the addition of 60 µg of the 32K protein was able to enhance film formation by the "natural" phospholipid obtained from lung almost to the level exhibited by the complex per se, it only moderately enhanced film formation using synthetic lipid. Similar results were obtained for addition of 13 µg of the 10K protein alone. However, when 13 µg of the 10K preparation was incubated with the synthetic phospholipid vesicles prior to the addition of 60 µg of 32K protein, film formation occurred at a rate and to a degree comparable to that of the natural complex per se. These results are shown in Figure 12.

## Claims (Claims for the following Contracting State(s): BE, CH, DE, FR, GB, IT, LI, LU, NL, SE)

1. A mammalian alveolar surfactant protein (ASP) in purified and isolated form which is:
32K ASP isolatable from human lung having the encoded amino acid sequence shown in Figure 3 herein;
an ASP isolatable from canine lung exhibiting an apparent molecular weight of about 16.5kd when determined on a non-reducing polyacrylamide gel and about 10-12kd when determined on a reducing polyacrylamide gel and having an amino acid sequence shown as the mature sequence in Figure 2 herein; or
an ASP isolatable from human lung exhibiting an apparent molecular weight of about 16.5kd when determined on a non-reducing polyacrylamide gel and about 10-11kd when determined on a reducing polyacrylamide gel and comprising the amino acid sequence shown in Figure 6 herein.

2. A recombinant DNA which encodes the ASP of claim 1.

3. A recombinant expression system capable, when contained in a recombinant host cell, of effecting the expression of a coding sequence encoding the ASP of claim 1, said expression system comprising a coding sequence for said ASP operably linked to control sequences capable of effecting its expression.

4. Recombinant host cells transformed with the expression system of claim 3 or the DNA of claim 2.

5. A method to produce ASP which comprises culturing the cells of claim 4 under conditions which favour the expression of said coding sequence so as to produce the encoded ASP; and
recovering the ASP produced from the cell culture.

6. A pharmaceutical composition effective in treating respiratory distress syndrome in mammals, which composition comprises the ASP of claim 1 or the ASP produced by the method of claim 5 in admixture with a pharmaceutically acceptable excipient.

7. The pharmaceutical composition of claim 6 wherein said excipient is a phospholipid vesicle preparation.

8. The ASP of claim 1 or the ASP produced by the method of claim 5 for use in treating respiratory distress syndrome.

## Claims (Claims for the following Contracting State(s): AT)

1. A method to produce alveolar surfactant protein (ASP) which is:
32K ASP isolatable from human lung having the encoded amino acid sequence shown in Figure 3 herein;
an ASP isolatable from canine lung exhibiting an apparent molecular weight of about 16.5kd when determined on a non-reducing polyacrylamide gel and about 10-12kd when determined on a reducing polyacrylamide gel and having an amino acid sequence shown as the mature sequence in Figure 2 herein; or
an ASP isolatable from human lung exhibiting an apparent molecular weight of about 16.5kd when determined on a non-reducing polyacrylamide gel and about 10-11kd when determined on a reducing polyacrylamide gel and comprising the amino acid sequence shown in Figure 6 herein;
which method comprises culturing host cells transformed with a recombinant expression system capable, of effecting the expression of a coding sequence encoding the ASP, said expression system comprising a coding sequence for said ASP operably linked to control sequences capable of effecting its expression and said culturing being under conditions which favour the expression of said coding sequence so as to produce the encoded ASP; and
which further comprises recovering the ASP produced from the cell culture.

2. A recombinant DNA which encodes the ASP defined in claim 1.

3. A recombinant expression system capable, when contained in a recombinant host cell, of effecting the expression of a coding sequence encoding the ASP defined in claim 1, said expression system comprising a coding sequence for said ASP operably linked to control sequences capable of effecting its expression.

4. Recombinant host cells transformed with the expression system of claim 3 or the DNA of claim 2.

5. A process for the preparation of a pharmaceutical composition effective in treating respiratory distress syndrome in mammals, which composition comprises the ASP produced by the method of claim 1 in admixture with a pharmaceutically acceptable excipient.

6. The process of claim 5 wherein said excipient is a phospholipid vesicle preparation.

## Patentansprüche (Patentansprüche für folgende(n) Vertragsstaat(en): BE, CH, DE, FR, GB, IT, LI, LU, NL, SE)

1. Ein alveoläres Surfactant-Protein aus Säugern (ASP) in gereinigter und isolierter Form, das ist:
aus menschlicher Lunge isolierbares 32 K ASP, das die hier in Figur 3 dargestellte codierte Aminosäuresequenz aufweist;
ein aus Hundelunge isolierbares ASP, das ein scheinbares Molekulargewicht von etwa 16.5 kd bei Bestimmung auf einem nichtreduzierenden Polyacrylamid-Gel und von etwa 10-12 kd bei Bestimmung auf einem reduzierenden Polyacrylamid-Gel zeigt und eine hier in Figur 2 als die reife Sequenz dargestellte Aminosäuresequenz aufweist; oder
ein aus menschlicher Lunge isolierbares ASP, das ein scheinbares Molekulargewicht von etwa 16.5 kd bei Bestimmung auf einem nichtreduzierenden Polyacrylamid-Gel und von etwa 10-11 kd bei Bestimmung auf einem reduzierenden Polyacrylamid-Gel zeigt, und die hier in Figur 6 dargestellte Aminosäuresequenz umfaßt.

2. Eine rekombinante DNA, die für das ASP aus Anspruch 1 codiert.

3. Ein rekombinantes Expressionssystem, das, wenn es in einer rekombinanten Wirtszelle enthalten ist, in der Lage ist, die Expression einer für das erwähnte ASP aus Anspruch 1 codierenden Codier-Sequenz zu bewirken, wobei das erwähnte Expressionssystem eine funktionell mit Kontrollsequenzen, die seine Expression bewirken können, verbundene Codiersequenz umfaßt.

4. Mit dem Expressionssystem aus Anspruch 3 oder der DNA aus Anspruch 2 transformierte rekombinante Wirtszellen.

5. Ein Verfahren zur Herstellung von ASP, das Kultivieren der Zellen aus Anspruch 4 unter Bedingungen, die die Expression der erwähnten Codiersequenz begünstigen, um das codierte ASP zu produzieren; und
Gewinnung des produzierten ASP aus der Zellkultur umfaßt.

6. Eine bei der Behandlung von Atemnotsyndrom bei Säugern wirksame pharmazeutische Zusammensetzung, wobei die Zusammensetzung das ASP aus Anspruch 1 oder das mit dem Verfahren aus Anspruch 5 produzierte ASP im Gemisch mit einem pharmazeutisch verträglichen Hilfsstoff umfaßt.

7. Die pharmazeutische Zusammensetzung aus Anspruch 6, wobei der erwähnte Hilfsstoff ein Phospholipid-Vesikel-Präparat ist.

8. Das ASP aus Anspruch 1 oder das durch das Verfahren aus Anspruch 5 hergestellte ASP zur Verwendung bei der Behandlung von Atemnotsyndrom.

## Patentansprüche (Patentansprüche für folgende(n) Vertragsstaat(en): AT)

1. Ein Verfahren zur Herstellung von alveolärem Surfactant-Protein (ASP), das ist:
aus menschlicher Lunge isolierbares 32 K ASP, das die hier in Figur 3 dargestellte codierte Aminosäuresequenz aufweist;
ein aus Hundelunge isolierbares ASP, das ein scheinbares Molekulargewicht von etwa 16.5 kd bei Bestimmung auf einem nichtreduzierenden Polyacrylamid-Gel und von etwa 10-12 kd bot Bestimmung auf einem reduzierenden Polyacrylamid-Gel zeigt und eine hier in Figur 2 als die reife Sequenz dargestellte Aminosäuresequenz aufweist; oder
ein aus menschlicher Lunge isolierbares ASP, das ein scheinbares Molekulargewicht von etwa 16.5 kd bei Bestimmung auf einem nichtreduzierenden Polyacrylamid-Gel und von etwa 10-11 kd bei Bestimmung auf einem reduzierenden Polyacrylamid-Gel zeigt, und die hier in Figur 6 dargestellte Aminosäuresequenz umfaßt,
wobei das Verfahren zur Herstellung des codierten ASP Kultivieren von mit einem rekombinanten Expressionssystem transformierten Wirtszellen umfaßt, das, wenn es in einer rekombinanten Wirtszelle enthalten ist, in der Lage ist, die Expression einer für das ASP aus Anspruch 1 codierenden Codierungs-Sequenz zu bewirken, wobei das erwähnte Expressionssystem eine funktionell mit Kontrollsequenzen, die seine Expression bewirken können, verbundene Codiersequenz umfaßt, wobei das erwähnte Kultivieren unter Bedingungen verläuft, die die Expression der erwähnten Codiersequenz begünstigen; und
das weiterhin die Gewinnung des produzierten ASP aus der Zellkultur umfaßt.

2. Eine rekombinante DNA, die für das in Anspruch 1 definierte ASP codiert.

3. Ein rekombinantes Expressionssystem, das, wenn es in einer rekombinanten Wirtszelle enthalten ist, in der Lage ist, die Expression einer für das in Anspruch 1 definierte ASP codierenden Codier-Sequenz zu bewirken, wobei das erwähnte Expressionssystem eine funktionell mit Kontrollsequenzen, die seine Expression bewirken können, verbundene Codiersequenz umfaßt.

4. Mit dem Expressionssystem aus Anspruch 3 oder der DNA aus Anspruch 2 transformierte rekombinante Wirtszellen.

5. Ein Verfahren zur Herstellung einer bei der Behandlung von Atemnotsyndrom bei Säugern wirksamen pharmazeutischen Zusammensetzung, wobei die Zusammensetzung das durch das Verfahren aus Anspruch 1 hergestellte ASP im Gemisch mit einem pharmazeutisch verträglichen Hilfsstoff umfaßt.

6. Das Verfahren aus Anspruch 5, wobei der erwähnte Hilfsstoff ein Phospholipid-Vesikel-Präparat ist.

## Revendications (Revendications pour l'(les) Etat(s) contractant(s) suivant(s): BE, CH, DE, FR, GB, IT, LI, LU, NL, SE)

1. Protéine de surfactant alvéolaire (ASP) de mammifère sous forme purifiée et isolée qui est :
un ASP 32K qui peut être isolé à partir du poumon humain, ayant la séquence d'acides aminés codée indiquée à la figure 3 ci-après ;
un ASP qui peut être isolé à partir du poumon de chien, présentant un poids moléculaire apparent d'environ 16,5 kd, lorsqu'il est déterminé sur gel de polyacrylamide non réducteur et environ 10-12 kd lorsqu'il est déterminé sur gel de polyacrylamide réducteur et ayant une séquence d'acides aminés indiquée sous forme de la séquence mature à la figure 2 ci-après ; ou
un ASP qui peut être isolé à partir du poumon humain, présentant un poids moléculaire apparent d'environ 16,5 kd lorsqu'il est déterminé sur gel de polyacrylamide non réducteur et environ 10-11 kd lorsqu'il est déterminé sur gel de polyacrylamide réducteur et comprenant la séquence d'acides aminés indiquée à la figure 6 ci-après.

2. ADN recombinant codant pour l'ASP de la revendication 1.

3. Système d'expression recombinant capable, lorsqu'il est contenu dans une cellule hôte recombinante, d'exprimer une séquence codante, codant pour l'ASP de la revendication 1, ledit système d'expression comprenant une séquence codant pour ledit ASP, liée de manière fonctionnelle à des séquences régulatrices capables d'assurer son expression.

4. Cellules hôtes recombinantes transformées par le système d'expression de la revendication 3 ou par l'ADN de la revendication 2.

5. Méthode de production d'ASP qui comprend la culture des cellules de la revendication 4 dans des conditions qui favorisent l'expression de ladite séquence codante de manière à produire l'ASP codé ; et
récupérer l'ASP produit à partir de la culture cellulaire.

6. Composition pharmaceutique efficace dans le traitement du syndrome de la détresse respiratoire chez les mammifères, composition qui comprend l'ASP de la revendication 1 ou l'ASP produit par la méthode de la revendication 5, en mélange dans un excipient pharmaceutiquement acceptable.

7. Composition pharmaceutique de la revendication 6 dans laquelle ledit excipient est une préparation de vésicules phospholipidiques.

8. ASP de la revendication 1 ou ASP produit par la méthode de la revendication 5 utilisable dans le traitement du syndrome de la détresse respiratoire.

## Revendications (Revendications pour l'(les) Etat(s) contractant(s) suivant(s): AT)

1. Méthode de production d'une protéine de surfactant alvéolaire (ASP) qui est :
un ASP 32K qui peut être isolé à partir du poumon humain, ayant la séquence d'acides aminés codée indiquée à la figure 3 ci-après ;
un ASP qui peut être isolé à partir du poumon de chien, présentant un poids moléculaire apparent d'environ 16,5 kd, lorsqu'il est déterminé sur gel de polyacrylamide non réducteur et environ 10-12 kd lorsqu'il est déterminé sur gel de polyacrylamide réducteur et qui possède une séquence d'acides aminés indiquée sous la forme de la séquence mature à la figure 2 ci-après ; ou
un ASP qui peut être isolé à partir du poumon humain présentant un poids moléculaire apparent d'environ 16,5 kd lorsqu'il est déterminé sur gel de polyacrylamide non réducteur et environ 10-11 kd lorsqu'il est déterminé sur gel de polyacrylamide réducteur et comprenant la séquence d'acides aminés indiquée à la figure 6 ci-après ;
méthode qui comprend la culture de cellules hôtes transformées par un système d'expression recombinant, capable d'exprimer une séquence codante codant pour l'ASP, ledit système d'expression comprenant une séquence codant pour ledit ASP, liée de manière fonctionnelle à des séquences régulatrices capables d'assurer son expression et ladite culture étant réalisée dans des conditions qui favorisent l'expression de ladite séquence codante de façon à produire l'ASP codé ; et
qui comprend en outre la récupération de l'ASP produit à partir de la culture cellulaire.

2. ADN recombinant codant pour l'ASP selon la revendication 1.

3. Système d'expression recombinant capable, lorsqu'il est contenu dans une cellule hôte recombinante, d'exprimer une séquence codante, codant pour l'ASP défini dans la revendication 1, ledit système d'expression comprenant une séquence codant pour ledit ASP, liée de manière fonctionnelle à des séquences régulatrices capables d'assurer son expression.

4. Cellules hôtes recombinantes transformées par le système d'expression de la revendication 3 ou par l'ADN de la revendication 2.

5. Procédé de préparation d'une composition pharmaceutique efficace dans le traitement du syndrome de la détresse respiratoire chez les mammifères, composition qui comprend l'ASP produit par la méthode de la revendication 1, en mélange dans un excipient pharmaceutiquement acceptable.

6. Procédé de la revendication 5 dans lequel ledit excipient est une préparation de vésicules phospholipidiques.
